# EUROPEAN PATENT SPECIFICATION

(11) **EP 4 547 231 B1**
(45) Date of publication and mention of the grant of the patent: **28.01.2026**
(21) Application number: 23736719.8
(22) Date of filing: 30.06.2023
(51) Int. Cl.: A61K 31/122, A61K 31/592, A61K 31/593, A61K 31/525, A61K 31/4415, A61K 31/519, A61P 9/10, A61K 9/00, A61K 31/14, A61K 31/197, A61K 31/202, A61K 31/375, A61K 31/4188, A61K 31/455, A61K 31/51, A61K 31/714, A61K 33/04, A61K 33/06, A61P 25/00, A61P 25/28

(54) **NUTRITIONAL COMPOSITION FOR STROKE**
ERNÄHRUNGSZUSAMMENSETZUNG FÜR SCHLAGANFALL
COMPOSITION NUTRITIONNELLE POUR UN ACCIDENT VASCULAIRE CÉRÉBRAL

(30) Priority: 01.07.2022 EP 22182684
(43) Date of publication of application: 07.05.2025
(73) Proprietor: N.V. Nutricia, 2712 HM Zoetermeer (NL)
(72) Inventor: BROERSEN, Ladislaus Maria, 3584 CT Utrecht (NL); SAVELKOUL, Paul Johannes Maria, 3584 CT Utrecht (NL); VAN WIJK, Nick, 3584 CT Utrecht (NL)
(74) Representative: Nederlandsch Octrooibureau
(86) International application number: PCT/EP2023/068080
(87) International publication number: WO 2024/003377

(56) References cited:
- WO-A1-2019/165222
- WO-A2-02/28379
- US-A1- 2014 314 731
- US-B1- 6 361 800
- DATABASE GNPD [online] MINTEL; 14 March 2022 (2022-03-14), ANONYMOUS: "Vanilla Flavour Complete Balanced Nutrition Supplement Powder Specially Designed for Sugar Management", XP093077507, retrieved from https://www.gnpd.com/sinatra/recordpage/9445884/ Database accession no. 9445884
- FERNÁNDEZ-VEGA BEATRIZ ET AL: "The Use of Vitamins and Coenzyme Q10 for the Treatment of Vascular Occlusion Diseases Affecting the Retina", NUTRIENTS, vol. 12, no. 3, 9 March 2020 (2020-03-09), pages 723, XP093002482, DOI: 10.3390/nu12030723
- CHELLUBOINA BHARATH ET AL: "Therapeutic potential of nutraceuticals to protect brain after stroke", NEUROCHEMISTRY INTERNATIONAL, ELSEVIER, AMSTERDAM, NL, vol. 142, 19 November 2020 (2020-11-19), XP086431495, ISSN: 0197-0186, [retrieved on 20201119], DOI: 10.1016/J.NEUINT.2020.104908
- DATABASE GNPD [online] MINTEL; 14 March 2022 (2022-03-14), ANONYMOUS: "Vanilla Flavour Complete Balanced Nutrition Supplement Powder Specially Designed for Sugar Management", XP093077507, retrieved from https://www.gnpd.com/sinatra/recordpage/9445884/ Database accession no. 9445884

## Description

### Field of the Invention

The present invention is in the field of compositions and nutritional composition for use in treating stroke, preferably the consequences of ischemic stroke. The invention further pertains to reducing the risk of recurrent stroke.

### Background

A stroke, also known as a cerebral vascular accident (CVA) is the rapid loss of brain function(s) due to disturbance in the blood supply to the brain. The World Health Organization (WHO) has defined stroke as, "rapidly developing clinical signs of focal (or global) disturbance of cerebral function, with symptoms lasting 24 hours or longer or leading to death, with no apparent cause other than of vascular origin". A stroke can be due to ischemia (lack of blood flow) caused by blockage of adequate blood flow to a section of the brain, usually caused by thrombus or other emboli lodging or forming in one of the blood vessels supplying the brain occurs resulting in ischemic stroke, or when a blood vessel bursts (or ruptures) as occurs in a haemorrhagic stroke.

Brain ischemia, also known as cerebral ischemia, is a resulting condition in which there is insufficient blood flow to the brain to meet metabolic demand. The ischemia may be focal ischemia, which is confined to a specific region of the brain, yet also global ischemia, which encompasses wide areas of brain tissue. If a blockage in ischemic stroke is not quickly resolved, the ischemia may cause the brain to become hypoxic leading to permanent neurologic damage or death. The consequences of strokes can be devastating and include impaired motor skills, impaired vision, and linguistic impairment. For example, the most common cause of ischemic stroke is occlusion of the middle cerebral artery (the intra-cranial artery downstream from the internal carotid artery), which damages cerebrum (e.g., cerebral cortex), e.g., the motor and sensory cortices of the brain. Such damage may result in hemiplegia, hemi-anaesthesia and, depending on the cerebral hemisphere damaged, either language or visuo-spatial deficits.

Despite treatment advances stroke is a main cause of death and disability worldwide, with one among every seven individuals suffering from stroke in their lifetime, and ischemic stroke accounting for more than 80% of strokes (Lackland et al., 2014). Effective treatments after stroke include reperfusion therapies, such as with recombinant tissue plasminogen activator; but this must be administered in a limited time window (within 4.5 hours of stroke onset); therefore, only 2-4% patients benefit from it (Hankey, 2017). Disability after stroke brings serious social and economic burdens; few people spontaneously recover and many are permanently disabled (Koh and Park, 2017). Traditional rehabilitation often has little effect (Feigin et al., 2017) with the lack of functional recovery in part being attributed to the restriction of regeneration and neuroplasticity of central nervous system neurons (Walmsley, A. R., and Mir, A. K. (2007) Current pharmaceutical design 13:2470-2484).

Numerous nutrients are further believed to be involved with supporting brain and neurological function. WO2019/074617 relates to multi-nutrient compositions enhancing the neurological function of a human through a.o. promoting neurite outgrowth. The multi-ingredient composition comprises high levels of vitamin K2 together with amongst others co-enzyme Q10, vitamin D3, cholecalciferol or ergocalciferol, and phosphatidyl choline.

Fernandez-Vega et al. Nutrients, 2020 relates to nutritional supplementation with different separate supplements including supplements with coenzyme Q10 (100 mg per day) and vitamin B2 (0.7 mg of vitamin B2 and 25 mg of coenzyme Q10 per day) for the treatment of vascular occlusion diseases affecting the retina, yet does not disclose a nutritional composition comprising a combination of coenzyme Q10 with both vitamin D and vitamin B2 for the treatment of the consequences of a stroke.

WO02/28379 A2 describes compositions and methods for lowering plasma lipoprotein(a) (Lp(a)) in humans and reducing the risk factors of a broad range of cardiovascular diseases. yet lacks an unambiguous disclosure of any combination of nutrient for use in the treatment of stroke. WO2019/165222 A1 describes a nutritional supplement of coQ10, vitamin B and vitamin D designed to support kidney and cardiac functions as well as mitochondrial energy function needs in patients undergoing haemodialysis yet does not disclose the treatment of (the consequences of) stroke. US2014/314731 A1 describes a composition consisting of Vitamin C, L-glutamine, L-cystine or L-cysteine, riboflavin, succinic acid, fumaric acid, coenzyme Q10, and niacin, and the use of this composition for minimizing the cell-damaging effect of radicals and harmful forms of oxygen, in particular of oxygen radicals or reactive oxygen species.

Chelluboina Barrath et al. Neurochemistry international, 2020 discloses the therapeutic potential of nutraceuticals to protect brain after stroke yet does not disclose the use of any specific combination nutraceuticals.

EP2938350 A1 discloses that an underlying cause of stroke is hypercholesterolemia which can be prevented by providing formulations that are able to lower LDL cholesterol blood levels, improve total cholesterol blood levels, increase HDL blood levels, lower triglyceride blood levels, and/or lower the systolic blood pressure. Said formulation comprises co-enzyme Q10, fish oil comprising eicosapentaenoic acid and docosahexaenoic acid, as well as vitamin D3.

XP093077507 *"Vanilla Flavour Complete Balanced Nutrition Supplement Powder Specially Designed for Sugar Management",* Database GNPD MINTEL, accession no. 9445884 discloses a nutritional supplement suitable for oral administration and tube feeding, comprising *inter alia*: CoQ10, vitamins B2, D3, choline and Polyunsaturated Fatty acids; as suitable heart-healthy lipid blend, but it is silent about stroke.

The ability of a damaged central nervous system (CNS) to regenerate after stroke is limited, so there remains an ongoing need in the art to find alternative ways to treat and promote recovery after stroke.

### Summary of the invention

The inventors surprisingly found that a composition comprising a combination of therapeutically effective amounts of co-enzyme Q10, vitamin D and vitamin B2 is effective in the treatment of stroke, in particular ischemic stroke. Treatment of stroke includes treatment of the consequences of a stroke. It was found in an *in vitro* model that a composition comprising a combination of co-enzyme Q10 and vitamin D, a combination of co-enzyme Q10 and vitamin B2, as well as a combination of co-enzyme Q10, vitamin D and vitamin B2 results in an increase of the outgrowth of neurites from surviving neurones after hypoxia and a reduction in neuronal damage after hypoxia as compared to without a composition comprising a combination of co-enzyme Q10 and vitamin D and/or vitamin B2. It was further observed that a combination of co-enzyme Q10 and vitamin D together with vitamin B2, B6 or B9 shows a beneficial improvement in neurite outgrowth after hypoxia. (i) Co-enzyme Q10 in combination with (ii) vitamin D and (iii) vitamin B2, together with (iv) polyunsaturated fatty acids and (v) choline, preferably phosphatidylcholine also beneficially increased outgrowth of neurites from surviving neurones after hypoxia and a reduction in neuronal damage.

The invention is defined in the appended set of claims.

The present invention thus concerns a nutritional composition comprising a combination of (i) coenzyme Q10, (ii) vitamin D, (iii) vitamin B2, (iv) polyunsaturated fatty acids and (v) choline, for use in treating stroke in a subject, preferably wherein the stroke is ischemic stroke, comprising administering to the subject a composition according to the invention. Preferably, the composition according to the invention further comprises one of more selected from A, B, C and E vitamin(s), phospholipids, selenium, and magnesium. In another preferred aspect the composition according to the invention comprises a combination of co-enzyme Q10, vitamin D, vitamin B2, polyunsaturated fatty acids, choline and one or more further B vitamins.

The invention may also be worded as a therapeutically effective combination of (i) co-enzyme Q10 with (ii) vitamin D and (iii) vitamin B2, and (iv) polyunsaturated fatty acids and (v) choline for the manufacture of a composition for treating stroke in a subject, preferably ischemic stroke. In other words, the invention concerns a composition for use in the treatment of stroke in a subject, preferably wherein the stroke is ischemic stroke, said composition comprising a combination of (i) co-enzyme Q10 with (ii) vitamin D and (iii) vitamin B2, (iv) polyunsaturated fatty acids and (v) choline.

In an embodiment, the invention particularly pertains to treating or preventing the consequences of stroke, preferably ischemic stroke, which involves an improvement (e.g., increase) in neurite length in treated subjects as compared with subjects not treated with the composition according to the invention. In some embodiments treatment of stroke, preferably ischemic stroke, further involves neuroprotection after stroke. In some embodiments the invention particularly pertains to treating or preventing the consequences of stroke, preferably ischemic stroke, which involves a prevention of or improvement in functional consequences of a stroke such as but not limited to hemiplegia, hemi-anaesthesia, language, or visuo-spatial deficits.

The invention further pertains to preventing or reducing the risk of a second or recurrent stroke in a patient, since patients having suffered from a first stroke ('first-ever stroke') are at increased risk of having another stroke within their lifetime. Within 5 years of a stroke, 24% of women and 42% of men will experience a recurrent stroke. Recurrent strokes often have a higher rate of death and disability because parts of the brain already injured by the original stroke may not be as resilient.

In a preferred embodiment, the administration or use of the composition is initiated after a stroke has occurred or has been diagnosed and the administration or use of the composition continues for a period of at least 2 weeks.

In a further preferred embodiment a daily supplementation of 5 to 90 mg of Co-enzyme Q10 with 5-80 ug of vitamin D, preferably vitamin D3 and 0.8 to 7.6 mg vitamin B2 is provided with the composition according to the invention.

### List of Figures

The present invention will be discussed in more detail below, with reference to the attached figures.
**Figure 1****.** The effects of co-enzyme Q10, vitamin D3, vitamin B2 and their combination on neurite outgrowth in primary neurons after hypoxia are shown. Neurite outgrowth is expressed as percentage relative to control (100%).
**Figure 2****.** The effects of co-enzyme Q10, vitamin D3, vitamin B6, vitamin B9 and their combination on neurite outgrowth in primary neurons after hypoxia are shown. Neurite outgrowth is expressed as percentage relative to control (100%).
**Figure 3****.** The effects of co-enzyme Q10, vitamin D3, vitamin B2, a PUFA and a source of choline and their combination on neurite outgrowth in an *in vitro* PC12 neuronal cell model. Neurite outgrowth is expressed as percentage relative to control (100%).
**Figure 4****.** The effect of different ratio's of choline chloride and phosphatidylcholine on neurite outgrowth in an *in vitro* PC12 neuronal cell model. Neurite outgrowth is expressed as percentage relative to control (100%).

### Detailed description of the invention

The present invention concerns a composition comprising a combination of (i) co-enzyme Q10 with (ii) vitamin D and (iii) vitamin B2, (iv) polyunsaturated fatty acids and (v) choline. In an embodiment the composition further comprises one or more of B6, and B9. Preferably, the composition according to the invention further comprises one of more selected from A, B, C and E vitamin(s), phospholipids, selenium, and magnesium.

The invention also concerns a composition comprising a combination of (i) coenzyme Q10 with (ii) vitamin Demd(iii) vitamin B2 (iv) polyunsaturated fatty acids and (v) choline for treating stroke in a subject, preferably ischemic stroke. Preferably, the composition according to the invention further comprises one of more selected from A, B, C and E vitamin(s), phospholipids, selenium, and magnesium.

Also, the invention pertains to a composition for use in the treatment of stroke in a subject, wherein the stroke is preferably ischemic stroke, said composition comprising a combination of (i) co-enzyme Q10 with (ii) vitamin D, (iii) vitamin B2, (iv) polyunsaturated fatty acids and (v) choline

Preferably, the composition according to the invention further comprises one of more selected from A, B, C and E vitamin(s), phospholipids, selenium, and magnesium. In a particular embodiment the composition comprises a combination of co-enzyme Q10 and vitamin D in combination with vitamin B2 and one or more of B6, and B9.

The invention further particularly pertains to treating stroke, more in particular treating or preventing the consequences of stroke, preferably ischemic stroke. The treatment of stroke, preferably ischemic stroke, involves an improvement (e.g., increase) in neurite length in subjects treated with the composition according to the invention as compared with subjects not treated with the composition according to the invention. In some embodiments, treatment of stroke, preferably ischemic stroke, further involves neuroprotection after stroke. The treatment of stroke, preferably ischemic stroke, further includes an improvement of post-hypoxic recovery of neurons and a reduction in inflammatory and oxidative processes. The treatment of stroke thus involves neuronal repair or regeneration and recovery of neuronal networks after a stroke has occurred. In an embodiment the treatment of stroke includes neuroprotection of neurons exposed to hypoxia. In an embodiment treatment of stroke, preferably ischemic stroke, further involves neuronal repair and/or recovery of neuronal networks. In some embodiments the invention particularly pertains to treating or preventing the consequences of stroke, preferably ischemic stroke, which involves a prevention of or improvement in functional consequences of a stroke such as but not limited to hemiplegia, hemi-anaesthesia, language, or visuo-spatial deficits. Without being bound by theory it is believed that the administration of co-enzyme Q10 together with vitamin D3 and vitamin B2 beneficially mitigates oxidative damage thereby aiding in reducing the extent of neuronal damage following hypoxia. It is further believed that vitamin B6 and B9 further enhances the antioxidant capacity of co-enzyme Q10 together with vitamin D3 and allows to mitigate oxidative damage and promote neuronal recovery. Polyunsaturated fatty acids in turn beneficially support brain metabolism, cell survival and provide anti-inflammatory action thereby supporting healing or recovery post hypoxia. Choline is associated with decreased neuroinflammation, improved functional and structural connectivity, had beneficial effects on cerebral blood flow. The ingredients of the composition according to the invention are believed to provide on one hand an optimal promotion of neurite outgrowth and recovery after hypoxia while at the same time supporting brain function.

The composition for use according to the invention is further described in the following embodiments.

### Definitions

Throughout this application, the following terminology and abbreviations may be used. "Nutritional composition" means a substance or formulation that satisfies at least a portion of a subject's nutrient requirements. The terms "nutritional(s)", "nutritional formula(s)", "enteral nutritional(s)", and "nutritional supplement(s)" are used as non-limiting examples of nutritional composition(s) throughout the present disclosure. Moreover, "nutritional composition(s)" may refer to liquids, powders, gels, pastes, solids, concentrates, suspensions, or ready-to-use forms of enteral formulas, oral formulas, formulas for infants, formulas for pediatric subjects, formulas for children, growing-up milks and/or formulas for adults.

The term "supplement" or "nutritional supplement" refers to a nutritional product that provides nutrients to an individual that may otherwise not conveniently be consumed in sufficient quantities by said individual and may be used to complement the nutrition of an individual. It may be in the form of tablets, capsules, pastilles or a liquid and the like. Supplements typically provide the selected nutrients while not representing a significant portion of the overall nutritional needs of the subject. Typically, they do not represent more than 0.1%, 1%, 5%, 10% of the daily energy need of the subject.

As used herein, "neuroprotection" refers to promotion of neuronal repair, limiting neuronal oxidative stress, limiting cell damage and/or promotion of recovery of neuronal networks after stroke.

"Prevention" includes reduction of risk and/or severity of neuronal damage from strokes. The terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition in a subject in need thereof.

The subject as used herein is a mammal, preferably a human.

As used herein, a "therapeutically effective amount" is an amount that reduces symptoms, manages progression of the consequences of stroke or provides a nutritional, physiological, or medical benefit to the individual.

The term "neurite" refers to processes growing out of a neuron. The term neurite as used herein encompasses all such cell processes (including both axons and dendrites) growing out of a neuron.

The term "neurite outgrowth" refers to the process of cells growing out of a neuron, or to the cells comprising an outgrowth from a neuron.

In this document and in its claims, the verb "to comprise" and its conjugations is used in its non-limiting sense to mean that items following the word are included, but items not specifically mentioned are not excluded. Further when used herein the term weight percent (wt%) is the percentage of weight based on the total weight. In addition, reference to an element by the indefinite article "a" or "an" does not exclude the possibility that more than one of the elements is present, unless the context clearly requires that there be one and only one of the elements. The indefinite article "a" or "an" thus usually means "at least one".

### Composition

The composition for use according to the invention involves administration of the composition according to the invention. The composition according to the invention may be used as a pharmaceutical product or a nutritional product. In one embodiment the present nutritional composition is a liquid. In one embodiment, preferably the present nutritional composition is a liquid ready-to-feed composition. Preferably the composition is administered orally or as tube feed.

In one aspect, the composition according to the invention may be used as a pharmaceutical product comprising one or more pharmaceutically acceptable carrier materials. Such product may contain the daily dosages as defined below in one or more dosage units. The dosage unit may be in a liquid form or in a solid form, wherein in the latter case the daily dosage may be provided by one or more solid dosage units, e.g. in one or more capsules or tablets. The pharmaceutical product, preferably for enteral application, may be a solid or liquid galenical formulation. Examples of solid galenical formulations are tablets, capsules (e.g. hard or soft shell gelatine capsules), pills, sachets, powders, granules and the like which contain the active ingredients together with conventional galenical carriers. Any conventional carrier material can be utilized. The carrier material can be organic or inorganic inert carrier material suitable for oral administration. Suitable carriers include water, gelatine, gum Arabic, lactose, starch, magnesium stearate, talc, vegetable oils, and the like. Additionally, additives such as thickeners, flavouring agents, preservatives, stabilizers, emulsifying agents, buffers and the like may be added in accordance with accepted practices of pharmaceutical compounding. While the individual active ingredients are suitably administered in a single composition, they may also be administered in individual dosage units.

In a preferred aspect, the composition according to the invention may be used as a nutritional product, for example as a nutritional supplement, e.g. as an additive to a normal diet, as a fortifier, to add to a normal diet, or as a complete nutrition. The nutritional product preferably comprises at least one component, preferably all components, selected from the group of fats, proteins, and carbohydrates. It is understood that a nutritional product differs from a pharmaceutical product by the presence of nutrients which provide nutrition to the subject to which the composition is administered, in particular the presence of protein, fat, digestible carbohydrates and dietary fibres. It may further contain ingredients such as minerals, vitamins, organic acids, and flavouring agents. Although the term "nutraceutical product" is often used in literature, it denotes a nutritional product with a pharmaceutical component or pharmaceutical purpose. Hence, the nutritional composition according to the invention may also be used in a nutraceutical product.

In one embodiment, the composition comprises a lipid fraction , wherein the lipid fraction provides between 15 and 50 energy% of the composition. In one embodiment, the composition is a liquid composition containing between 1.1 and 2.6 kcal per ml, preferably between 1.2 and 2.4 kcal per ml. In one embodiment the composition is a tube feed having a caloric density of between 1.1 and 1.7 kcal per ml, preferably between 1.2 and 1.6 kcal per ml. In another embodiment the composition is in the form of an oral nutritional supplement having a caloric density of between 2.2 and 2.6 kcal per ml. The energy provided by nutrients is calculated using the Atwater calculation factors of 9 kcal per g lipid, 4 kcal per gram protein or gram digestible carbohydrate, 2 kcal per gram fiber and zero kcal for the other components in the product.

The amount of lipid fraction can be determined by applying the methods known in the art for measuring fat content in the food matrix as applicable. For example, fat content for general foods is determined by applying AOAC(R) official method 983.23, while the Roese-Gottlieb method (AOAC(R) 932.06) is better applicable for products based on dried milk (Lehner, R., Estoppey, A., (1954) Mitt. Lebensmitteluntersuchung Hyg. 54:183-185). The amount of individual lipid components can be determined by applying methods specifically designed for measuring that specific component or by fractionating the fat fraction isolated from the extraction of the chloroform-niethanol fraction as given in the 983.23 method.

Suitable sources of lipids for use in the composition include but are not limited to fish oil, krill oil, algae oil, soybean oil, rape seed oil (such as colza oil, low erucic acid rape seed oil and canola oil), high oleic sunflower oil, coconut oil, high oleic safflower oil and olive oil. An alternative option are lipids obtained from milk from non-human mammals, preferably cow's milk, goat milk, sheep milk, horse milk, buffalo milk, yak milk, reindeer milk, donkey milk and camel milk, particularly cow's milk and/or goat milk. Milk lipid is sometimes also referred to as milk fat or butter fat.

The nutritional composition according to the invention thus preferably comprises a lipid fraction, preferably a lipid fraction suitable for nutrition as known in the art. The composition comprises 4 to 12 g lipid per 100 ml, preferably 4.5 to 11 g lipid per 100 ml, more preferably 5 to 10 g per 100 ml. Based on calories, preferably the compostion according to the invention may comprise 3 to 5.5 g of lipid per 100 kcal, preferably 3.5 to 5 g per 100 kcal, more preferably 3.8 to 4.5 g of lipid per 100 kcal based on the total energy content of the composition. The lipid fraction includes polyunsaturated fatty acids and optionally also mono-unsaturated fatty acids (MUFAs) and saturated fatty acids (SFA).

The composition according to the invention preferably comprises 4 to 17 g of protein per 100 ml of the composition. The total protein that is present in the nutritional composition, i.e. the combination of all proteins present, may also be referred to as the "protein fraction" of the nutritional composition. The nutritional composition thus preferably comprises a protein fraction of 4 - 17 g per 100 ml of the composition. In a preferred aspect the nutritional composition comprises a protein fraction of 5 to 16 g per 100 ml of the composition, more preferably of 6 - 15 g. Most preferably the composition comprises a protein fraction of 11 to 14.5 g per 100 ml of the composition. Based on calories, preferably the compostion according to the invention may comprise 4 to 7 g of protein fraction per 100 kcal, preferably 4.5 to 6.5 g of protein fraction per 100 kcal, more preferably 5 to 6 g of protein fraction per 100 kcal based on the total energy content of the composition.

Suitable protein sources may be based on cows' milk proteins such as whey, casein and mixtures thereof and vegetable proteins such as those based on soy, potato, pea and the like.The nutritional composition preferably further comprises a carbohydrate fraction. The composition preferably comprises 9 to 25 g carbohydrates per 100 ml, more preferably 10 to 24 g per 100 ml, even more preferably 11 to 23 per 100 ml. Based on calories, preferably the compostion according to the invention may comprise 8.5 to 10.5 g of carbohydrates per 100 kcal, preferably 9 to 10 g per 100 kcal, more preferably 9.2 to 9.6 g of carbohydrates per 100 kcal based on the total energy content of the composition.

Suitable carbohydrate sources include lactose, glucose, sucrose, fructose, galactose, maltose, starch, isomaltulose, and maltodextrin.

The composition of the invention is typically an enteral composition, i.e. intended for oral administration such as a tube feed, a nutritional drink and an oral nutritional supplement. It is preferably administered in liquid form. Preferably, the composition comprises water in which the further components are dissolved or suspended.

The composition is thus preferably a liquid, or a solid (typically a powder or tablet, preferably a powder) which is reconstitutable with a liquid, preferably with water, to obtain a liquid composition. Dosages of components defined below may for example be in daily dose or in a concentration per 100 mL or 100 kcal.

The composition according to the invention is for treating stroke, preferably ischemic stroke. The stroke is preferably an ischemic stroke, which may also be referred to as ischemic event, ischemic episode, ischemic attack or cerebral infarction and the treatment particularly involves treatment of the consequences of a stroke. The treatment involves an improvement in neurite outgrowth.

The compositions as described above can be used as a nutritional therapy, nutritional support, as a medical food, as a food for special medical purposes or as a nutritional supplement. Administration of the composition according to the invention typically occurs during recovery and/or rehabilitation after the occurrence of a stroke, and may be continued as long as negative effects thereof prolong. Although positive effects are already observed during the first week of administration, administration is preferably continued for at least 2 weeks, more preferably at least 4 weeks, even more preferably at least 8 weeks. Administration preferably starts at the first day after occurrence of a stroke. The composition according to the invention can be consumed in the form of a liquid composition, preferably an oral nutritional supplement at one, two or three servings of 50 - 250 mL per day, typically servings of 125 mL each, during recovery and/or rehabilitation after the occurrence of a stroke. Preferred daily dosages are in the range of 100 to 500 mL, more preferably 125 to 375 mL, most preferably 200 to 300 mL. Preferably, the composition is enterally administered. Administration occurs preferably at least one time per day, although alternative dosage regimes can be determined from these numbers. Alternatively the composition may be a tube feed and is administered continuously. Preferred daily dosages of a tube feed are in the range of 1000 to 3000 kcal per day, preferably 1500 kcal to 2500 kcal per day. Preferably about 1 to 2 liters of tube feed is administered per day.

The composition for use according to the invention relates to composition comprising a combination of (i) co-enzyme Q10 with (ii) vitamin D, (iii) vitamin B2, (iv) polyunsaturated fatty acids and (v) choline In an embodiment the composition further comprises one or more of vitamins B6, and B9. Preferably, the composition according to the invention further comprises one of more selected from A, further B, C, and E vitamin(s), phospholipids, selenium and magnesium.

### Co-enzyme Q10

The composition according to the invention comprises therapeutically effective amounts of Co-enzyme Q10.

Co-enzyme Q10 (also known as CoQ10, Q10, vitamin Q10, ubiquinone, and ubidecarenone) is a benzoquinone compound synthesized naturally by the human body. The "Q" and the "10" in the name refer to the quinone chemical group and the 10 isoprenyl chemical subunits, respectively, that are part of this compound's structure. Co-enzyme Q10 as used herein refers to ubiquinone (2,3 dimethoxy-5 methyl-6-decaprenyl benzoquinone) and/or its functional analogues including ubiquinol.

Co-enzyme Q10 is a natural substance soluble in fats that plays a main role as an enzyme cofactor necessary in the transporting chain of mitochondrial electrons, and it is key factor in oxidative phosphorylation and aerobe cell breathing by generating adenosine triphosphate (ATP) (Egil Fosslien; Mitochondrial Medicine - Molecular Pathology of Defective Oxidative Phosphorylation, Annals of Clinical and Laboratory Science, 2000).

CoQ10 was proven to act both as an antioxidant and as an electron carrier due to its lipid solubility and its capacity to exist in both completely reduced forms (ubiquinol) and completely oxidized forms (ubiquinone).

CoQ10 accepts electrons derived from both complex I (NADH ubiquinone oxidoreductase) and complex II (succinate ubiquinone reductase), and then transports them to complex III (ubiquinol cytochrome c reductase).

Natural sources of CoQ10 and/or analogues that are suitable for this invention include extracts or isolates of fungi, yeasts or bacteria, canola oil or soybean oil.

The present composition preferably comprises 1 to 15 mg Co-enzyme Q10 per 100 ml, preferably 1.5 to 14 mg Co-enzyme Q10 per 100 ml, more preferably 2 to 13 mg Co-enzyme Q10 per 100 ml, even more preferably 2.5 to 12 mg Co-enzyme Q10 per 100 ml composition.

According to one embodiment Co-enzyme Q10 is preferably provided in an amount of 5 to 90 mg per day, more preferably 8 to 80 mg per day, even more preferably 10 to 70 mg per day, most preferably 15 to 60 mg per day.

In an embodiment the composition is in the form of a tube feed and Co-enzyme Q10 is preferably provided in an amount of 8 to 75 mg per day, more preferably 10 to 70 mg per day, even more preferably 15 to 65 mg per day, most preferably 20 to 60 mg per day.

In another embodiment when the composition is in the form of an oral nutritional supplement Co-enzyme Q10 is preferably provided in an amount of 8 to 60 mg per day, more preferably 10 to 55 mg per day, even more preferably 12 to 50 mg per day, most preferably 15 to 45 mg per day.

Based on calories, preferably the nutrional compostion according to the invention comprises 1.5 to 5.5 mg Co-enzyme Q10 per 100 kcal, preferably 1.8 to 5.2 mg Co-enzyme Q10 per 100 kcal, more preferably 2 to 5 mg Co-enzyme Q10 per 100 kcal based on the total energy content of the nutritional composition. The above numbers are based on the molar weight of ubiquinone.

### Vitamin D

The present composition comprises vitamin D.

Vitamin D is a group of fat-soluble secosteroids, the two major physiologically relevant forms of which are vitamin D2 (ergocalciferol) and vitamin D3 (cholecalciferol). These are known collectively as calciferol. Vitamin D without a subscript refers to all forms of vitamin D, either D1, D2, D3, or D4, in particular D2 and D3, or any mixture thereof. The composition according to the invetion preferably comprises vitamin D3.

When ingested, vitamin D is hydroxylated in the liver (endoplasmic reticulum) to 25-hydroxycholecalciferol (25(OH)D), also known as calcidiol, by the enzyme 25-hydroxylase, produced by hepatocytes. Once made, the product is stored in the hepatocytes until it is needed and can be released into the plasma where it will be bound to an α-globulin. 25-hydroxycholecalciferol is then transported to the proximal tubules of the kidneys where it can be hydroxylated by one of two enzymes to different forms of vitamin D, one of which is active vitamin D (1,25(OH)₂D) and another which is inactive vitamin D (24,25(OH)D). The enzyme 1α-hydroxylase which is activated by parathyroid hormone (and additionally by low calcium or phosphate) forms the main biologically active vitamin D hormone with a C1 hydroxylation forming 1,25- dihydroxycholecalciferol (1,25(OH)₂D, also known as calcitriol). A separate enzyme hydroxylates the C24 atom forming 24R,25(OH)₂D3 when 1α-hydroxylase is not active, this inactivates the molecule from any biological activity.

Vitamin D may be provided in an active (1,25(OH)₂D) or unactive (Vit D3 or D2) form.

The present composition preferably comprises 1.5 to 10 µg vitamin D, preferably 2 to 9 µg vitamin D per 100 ml, more preferably 2.5 to 8 µg vitamin D per 100 ml composition.

According to one embodiment, vitamin D, preferably vitamin D3 is supplemented in an amount of 5 to 80 µg per day, preferably 6 to 75 µg per day, more preferably 8 to 70 µg per day, even more preferably 10 to 65 µg per day.

In an embodiment the composition is in the form of a tube feed and vitamin D, preferably vitamin D3, is preferably provided in an amount of 14 to 80 µg per day, more preferably 16 to 75 µg per day, even more preferably 18 to 70 µg per day, most preferably 20 to 65 µg per day.

In another embodiment when the composition is in the form of an oral nutritional supplement vitamin D, preferably vitamin D3 is preferably provided in an amount of 5 to 45 µg per day, more preferably 6 to 40 µg per day, even more preferably 8 to 35 µg per day, most preferably 10 to 30 µg per day.

Based on calories, preferably the nutrional compostion according to the invention comprises 1.3 to 4.5 µg vitamin D, preferably vitamin D3, per 100 kcal, preferably 1.5 to 4 µg vitamin D per 100 kcal, more preferably 2 to 3.5 µg per 100 kcal based on the total energy content of the nutritional composition. The above numbers are based on the molar weight of vitamin D3.

### Vitamin B

The present composition comprises vitamin B2 and may comprise other B-vitamins. B-vitamins are known as nutritional co-factors that act as biochemical "spark plugs" in mitochondria, and therefore act to further nurture and support optimal neural metabolism.

The present composition comprising B2, further preferably comprises at least one vitamin B and preferably at least two B vitamins selected from the group of vitamin B1 (thiamine), B3 (niacin or niacinamide), vitamin B5 (pantothenic acid), B6 (pyridoxine, pyridoxal, or pyridoxamine, or pyridoxine hydrochloride), vitamin B7 (biotin) vitamin B9 (folic acid or folate), and vitamin B12 (cobalamins). Functional equivalents are encompassed within these terms. The term "vitamin B12" incorporates all cobalamin equivalents known in the art.

Preferably, further to B2, the B vitamins in the context of the composition according to the invention comprises at least one, preferably at least two even more preferably at least three selected from the group of vitamin B1, B3, B5, B6, B7, vitamin B12 and vitamin B9. More preferably the composition comprises at least one of vitamin B6 and/or B9. In a further preferred embodiment the composition comprisesone or more of vitamin B6 and/or B9, preferably vitamin B6 and B9, more preferably vitamin B6, B9 and B12, even more preferably vitamin B1, vitamin B6, vitamin B9 and vitamin B12 . In one embodiment the composition according to the invention may include all of vitamin B1 (thiamine), B6 (pyridoxine, pyridoxal, or pyridoxamine, or pyridoxine hydrochloride), vitamin B9 (folic acid or folate), and vitamin B12 (cyanocobalamin). In some embodiment the composition may comprise further B vitamins, further B vitamins as used herein are vitamin B1, B3, B5 and B7 which the composition may comprise in addition to the one or more vitamin B6 and vitamin B9.

If present in the composition, the vitamin B1 is preferably present in an amount to provide a daily dosage in the range of 0.7 to 5 mg, preferably in the range of 0.8 to 4.9 mg, more preferably in the range of 0.9 to 4.8 mg. When the composition is in the form of a tube feed the daily amount of vitamin B1 administered is preferably 1.2 to 5.5 mg per day, more preferably 1.5 to 5 mg per day, most preferably 1.6 to 4.8 mg per day. When the composition is in the form of a oral nutritional supplement the daily amount of vitamin B1 administered is preferably 0.7 to 2.9 mg per day, more preferably 0.8 to 2.8 mg per day, most preferably 0.9 to 2.7 mg per day. If present,the composition preferably comprises 0.1 to 0.9 mg vitamin B1 per 100 ml, more preferably 0.15 to 0.8 mg vitamin B1 per 100 ml, more preferably 0.2 to 0.75 mg vitamin B1 per 100 ml. Based on calories, preferably the nutrional compostion according to the invention may comprise 0.05 to 0.4 mg vitamin B1 per 100 kcal, preferably 0.1 to 0.35 mg vitamin B1 per 100 kcal, more preferably 0.15 to 0.30 vitamin B1 per 100 kcal based on the total energy content of the composition. The above numbers are based on the molar weight of thiamin.

Vitamin B2 or riboflavin is a starting compound in the synthesis of the co-enzymes flavin mononucleotide (FMN, also known as riboflavin-5'-phosphate) and flavin adenine dinucleotide (FAD). The latter co-enzymes are critically involved in energy metabolism, cellular respiration, growth and development as well as co-factors in the metabolism of essential fatty acids in brain lipids. A high proportion (>50%) of acute ischemic stroke patients has been reported to be riboflavin deficient as indicated by high red blood cell glutathione reductase activation coefficient (EGRAC) (Gariballa, S. et al. Eur J Clin Nutr, 2007).

The vitamin B2 is preferably present in an amount to provide a daily dosage in the range of 0.8 to 7.6 mg, preferably in the range of 1to 7.4 mg, more preferably in the range of 1.2 to 7.2 mg per day. When the composition is in the form of a tube feed the daily amount of vitamin B2 administered is preferably 2 to 7.6 mg per day, more preferably 2.2 to 7.4 mg per day, most preferably 2.4 to 7.2 mg per day. When the composition is in the form of a oral nutritional supplement the daily amount of vitamin B2 administered is preferably 0.8 to 4 mg per day, more preferably 1 to 3.8 mg per day, most preferably 1.2 to 3.6 mg per day. If present, thecomposition preferably comprises 0.1 - 1.4 mg vitamin B2 per 100 ml, more preferably 0.2 - 1.2 mg vitamin B2 per 100 ml, more preferably 0.3 - 1 mg vitamin B2 per 100 ml. Based on calories, preferably the nutrional compostion according to the invention may comprise 0.4 to 0.6 mg vitamin B2 per 100 kcal, preferably 0.15 to 0.5 mg vitamin B2 per 100 kcal, more preferably 0.2 to 0.4 mg vitamin B2 per 100 kcal based on the total energy content of the composition. The above numbers are based on the molar weight of riboflavin.

If present in the composition, the vitamin B3 is preferably present in an amount to provide a daily dosage in the range of 3 to 45 mg, preferably in the range of 3.5 to 42 mg, more preferably in the range of 3.8 to 40 mg niacin equivalent per day. When the composition is in the form of a tube feed the daily amount of vitamin B3 administered is preferably 11 to 42 mg per day, more preferably 12 to 40 mg per day, most preferably 12.5 to 39 mg niacin equivalents per day. When the composition is in the form of a oral nutritional supplement the daily amount of vitamin B3 administered is preferably 3.4 to 20 mg per day, more preferably 3.6 to 19 mg per day, most preferably 3.8 to 18 mg niacin equivalents per day. If present, thecomposition preferably comprises 1.2 to 6 mg niacin equivalent per 100 ml, more preferably 1.4 to 5.5 mg niacin equivalent per 100 ml, more preferably 1.6 to 5 mg niacin equivalent per 100 ml. Based on calories, preferably the nutrional compostion according to the invention may comprise 1 to 2.5 mg niacin equivalent per 100 kcal, preferably 1.1 to 2.2 mg niacin equivalent per 100 kcal, more preferably 1.2 to 2 mg niacin equivalent per 100 kcal based on the total energy content of the composition. The above numbers are based on the molar weight of niacin.

If present in the composition, the vitamin B5 is preferably present in an amount to provide a daily dosage in the range of 0.8 to 15 mg, preferably in the range of 1 to 14 mg, more preferably in the range of 1.2 to 13 mg per day. When the composition is in the form of a tube feed the daily amount of vitamin B5 administered is preferably 3.5 to 14 mg per day, more preferably 4 to 13 mg per day, most preferably 4.1 to 12.6 mg per day. When the composition is in the form of a oral nutritional supplement the daily amount of vitamin B5 administered is preferably 0.8 to 7.5 mg per day, more preferably 1 to 7 mg per day, most preferably 1.2 to 6.8 per day. If present, thecomposition preferably comprises 0.3 to 2.2 mg vitamin B5 per 100 ml, more preferably 0.4 to 2 mg vitamin B5 per 100 ml, more preferably 0.5 to 1.8 mg vitamin B5 per 100 ml. Based on calories, preferably the nutrional compostion according to the invention may comprise 0.2 to 1 mg vitamin B5 per 100 kcal, preferably 0.3 to 0.9 mg vitamin B5 per 100 kcal, more preferably 0.4 to 0.8 mg vitamin B5 per 100 kcal based on the total energy content of the composition. The above numbers are based on the molar weight of panthotenic acid.

The active form of vitamin B6, pyridoxal 5'-phosphate (PLP), serves as a co-enzyme in multiple enzyme reactions in amino acid, glucose, and lipid metabolism and is known to be the rate limiting factor in the synthesis of some neurotransmitters.

If present in the composition, the vitamin B6 is preferably present in an amount to provide a daily dosage in the range of 0.7 to 9 mg, preferably in the range of 0.8 to 8 mg, more preferably in the range of 0.9 to 7.5 mg. When the composition is in the form of a tube feed the daily amount of vitamin B6 administered is preferably 1.5 to 9 mg per day, more preferably 2 to 8 mg per day, most preferably 2.5 to 7.5 mg per day. When the composition is in the form of a oral nutritional supplement the daily amount of vitamin B6 administered is preferably 0.7 to 3.5 mg per day, more preferably 0.8 to 3 mg per day, most preferably 0.9 to 2.7 mg per day. If present, thecomposition preferably comprises 0.2 to 1mg vitamin B6 per 100 ml, more preferably 0.25 - 0.8 mg vitamin B6 per 100 ml, more preferably 0.3 to 0.7 mg vitamin B6 per 100 ml. Based on calories, preferably the compostion according to the invention may comprise 0.1 to 0.5 mg vitamin B6 per 100 kcal, preferably 0.2 to 0.4 mg vitamin B6 per 100 kcal, more preferably 0.25 to 0.3 mg vitamin B6 per 100 kcal based on the total energy content of the composition. The above numbers are based on the molar weight of pyridoxine.

If present in the composition, the vitamin B7 is preferably present in an amount to provide a daily dosage in the range of 8 to 95 µg, preferably in the range of 9 to 90 µg, more preferably in the range of 9.5 to 85 µg per day. When the composition is in the form of a tube feed the daily amount of vitamin B7 administered is preferably 24 to 95 µg per day, more preferably 26 to 90 µg per day, most preferably 28 to 88 µg per day. When the composition is in the form of a oral nutritional supplement the daily amount of vitamin B7 administered is preferably 9.4 to 45 µg per day, more preferably 9.6 to 42 µg per day, most preferably 9.8 to 41 µg per day. If present, thecomposition preferably comprises 2 to 14 µg vitamin B7 per 100 ml, more preferably 3 to 12 µg vitamin B7 per 100 ml, more preferably 3.5 to 11 µg vitamin B7 per 100 ml. Based on calories, preferably the compostion according to the invention may comprise 1.5 to 6 µg vitamin B7 per 100 kcal, preferably 2 to 5.5 µg vitamin B7 per 100 kcal, more preferably 2.5 to 5 µg vitamin B7 per 100 kcal based on the total energy content of the composition. The above numbers are based on the molar weight of biotin.

Vitamin B9 or folic acid is an essential nutrient that id converted by the body into folate, which is required for DNA and RNA biosynthesis, DNA repair and one-carbon metabolic reactions. Decreased folate levels have frequently been reported in stroke patients and have been associated with hyperhomocysteinemia which is common in stroke patients (Poddar, R, Exp Neurol, 2021).

If present in the composition, the vitamin B9 is preferably present in an amount to provide a daily dosage in the range of 150 to 900 µg, preferably in the range of 175 to 800 µg, more preferably in the range of 200 to 750 µg per day. When the composition is in the form of a tube feed the daily amount of vitamin B9 administered is preferably 200 to 800 µg per day, more preferably 225 to 775 µg per day, most preferably 240 to 750 µg per day. When the composition is in the form of a oral nutritional supplement the daily amount of vitamin B9 administered is preferably 150 to 700 µg per day, more preferably 175 to 650 µg per day, most preferably 200 to 600 µg per day. If present, thecomposition preferably comprises 20 to 225 µg vitamin B9 per 100 ml, more preferably 25 to 200 µg vitamin B9 per 100 ml, more preferably 30 to 175 µg vitamin B9 per 100 ml. Based on calories, preferably the compostion according to the invention may comprise 16 to 80 µg vitamin B9 per 100 kcal, preferably 18 to 75 µg vitamin B9 per 100 kcal, more preferably 20 to 70 µg vitamin B9 per 100 kcal based on the total energy content of the composition. The above numbers are based on the molar weight of folic acid.

If present in the composition, the vitamin B12 is preferably present in an amount to provide a daily dosage in the range of 2 to 20 µg, preferably in the range of 2.5 to 19 µg, more preferably in the range of 3 to 18 µg per day. When the composition is in the form of a tube feed the daily amount of vitamin B9 administered is preferably 4 to 22 µg per day, more preferably 5 to 20 µg per day, most preferably 6 to 18 µg per day. When the composition is in the form of a oral nutritional supplement the daily amount of vitamin B9 administered is preferably 2 to 12 µg per day, more preferably 2.5 to 10 µg per day, most preferably 3 to 9 µg per day. If present, thecomposition preferably comprises 0.5 to 3 µg vitamin B12 per 100 ml, more preferably 0.6 to 2.6 µg vitamin B12 per 100 ml, more preferably 0.7 to 2.4 µg vitamin B12 per 100 ml. Based on calories, preferably the compostion according to the invention may comprise 0.4 to 1.4 µg vitamin B12 per 100 kcal, preferably 0.5 to 1.2 µg vitamin B12 per 100 kcal, more preferably 0.6 to 1 µg vitamin B12 per 100 kcal based on the total energy content of the composition. The above numbers are based on the molar weight of cyanocobalamin.

### n-3 PUFA

The present composition comprises polyunsaturated fatty acids, preferably omega-3 polyunsaturated fatty acids (PUFA), or n-3 LC-PUFA (long-chain PUFAs).

The composition comprises at least one, preferably two omega-3 long-chain polyunsaturated fatty acids (LC-PUFA; having a chain length of 18 and more carbon atoms) selected from the group consisting of docosahexaenoic acid (22:6, ω-3; DHA), eicosapentaenoic acid (20:5, ω-3; EPA) and docosapentaenoic acid (22:5 w-3; DPA), preferably DHA and EPA.

EPA is converted to DPA (w-3), increasing subsequent conversion of DPA to DHA in the brain. The present composition preferably contains a significant amount of EPA, so to further stimulate *in vivo* DHA formation.

The DHA, EPA and/or DPA may be provided in any form such as, but not limited to, triglycerides, diglycerides, monoglycerides, free fatty acids or their salts or esters, phospholipids, lysophospholipids, glycerol ethers, lipoproteins, ceramides, glycolipids or combinations thereof. Preferably, the present composition comprises at least DHA in triglyceride form.

Suitable ω-3 LCPUFA and/or sources of DHA and EPA include tuna oil, (other) fish oils, DHA-rich alkyl esters, algae oil, egg yolk, krill oil or phospholipids enriched with ω-3 LCPUFA e.g. phosphatidylserine-DHA. Preferably, the composition or composition for use according to the invention comprises fish oil providing the omega-3 LCPUFA(s). Another particularly suitable source for the omega-3 LCPUFA(s) is algae oil.

DHA is preferably administered in an amount of 100 to 1250 mg per day, more preferably 150 to 1200 mg per day, even more preferably 200 to 1100 mg per day, most preferably 250 to 1000 mg per day.

In an embodiment when the composition is in the form of a tube feed the daily amount of DHA administered is preferably 200 to 1100 mg per day, more preferably 250 to 1050 mg per day, most preferably 300 to 1000 mg per day.

In another embodiment when the composition is in the form of a oral nutritional supplement the daily amount of DHA administered is preferably 150 to 850 mg per day, more preferably 200 to 800 mg per day, most preferably 250 to 750 mg per day.

The present composition preferably comprises 30 to 240 mg DHA per 100 ml of the composition, preferably 35 to 220 mg DHA per 100 ml, more preferably 40 to 200 mg DHA per 100 ml composition.

Based on calories, preferably the nutrional compostion according to the invention comprises 20 to 100 mg DHA per 100 kcal, preferably 25 to 90 mg DHA per 100 kcal, more preferably 30 to 80 mg DHA per 100 kcal based on the total energy content of the nutritional composition. The above numbers are based on the molar weight of DHA.

EPA is preferably administered in an amount of 300 to 2400 mg per day, more preferably 400 to 2200 mg per day, most preferably 500 to 2000 mg per day.

In an embodiment when the composition is in the form of a tube feed the daily amount of EPA administered is preferably 500 to 2300 mg per day, more preferably 600 to 2100 mg per day, most preferably 650 to 2000 mg per day.

In another embodiment when the composition is in the form of a oral nutritional supplement the daily amount of EPA administered is preferably 300 to 1750 mg per day, more preferably 400 to 1600 mg per day, most preferably 500 to 1500 mg per day.

The present composition preferably comprises 70 to 440 mg EPA per 100 ml of the composition, preferably 80 to 420 mg EPA per 100 ml, more preferably 90 to 400 mg EPA per 100 ml composition. Based on calories, preferably the nutrional compostion according to the invention comprises 50 to 225 mg EPA per 100 kcal, preferably 60 to 200 mg EPA per 100 kcal, more preferably 70 to 170 mg EPA per 100 kcal based on the total energy content of the nutritional composition. The above numbers are based on the molar weight of EPA.

The weight ratio of DHA to EPA is preferably lower than 1, more preferably 1:1.1 to:1:4, more preferably 1:1.3 to 1:2.5. In some embodiments de weight ratio of DHA to EPA is about 1:2.

In terms of daily dosage, the present use and method preferably comprises the administration of 400 to 3750 mg n-3 LC-PUFAs (preferably DHA+EPA+DPA, most preferably DHA+EPA) per day, more preferably 550 to 3500 mg per day, even more preferably 650 to 3250 mg per day, most preferably 750 to 3000 mg per day.

The present composition preferably comprises of n-3 LC-PUFA (more preferably DHA+EPA+DPA, most preferably DHA+EPA) on the total fatty acids is preferably 1 to 8 wt%, more preferably 1.5 to 7.5 wt%, most preferably 2 to 7 wt%.

The present composition preferably comprises 0.1 to 3.5 wt% DHA based on total fatty acids, preferably 0.3 to 3 wt% DHA based on total fatty acids, more preferably 0.5 to 2.5 wt% DHA based on total fatty acids. The present composition preferably comprises 0.5 to 6 wt% EPA based on total fatty acids, preferably 1 to 5 wt% EPA, most preferably 1.5 to 4.5 wt%, based on total fatty acids.

The above-mentioned ratios and amounts take into account and optimise several aspects, including taste (too high n-3 LC-PUFA levels reduce taste, resulting in a reduced compliance), balance between DHA and precursors thereof to ensure optimal effectiveness while maintaining low-volume formulations.

### Choline

The present composition comprises choline. Choline may be present as such, or as choline equivalent in the form of a choline salt and/or choline ester. The choline salt is preferably selected from choline chloride, choline bitartrate, or choline stearate. The choline ester is preferably selected from a phosphatidylcholine and lyso-phosphatidyl choline, preferably phosphatidylcholine. In a preferred embodiment the present composition comprises a) choline as such or a choline salt and b) a choline ester, preferably phosphatidylcholine. This means that the present composition preferably comprises both choline as such or a choline salt and in addition a choline ester, preferably phosphatidylcholine.

The present composition for use preferably comprises the administration of more than 70 mg of a) choline or choline salt per day, preferably 70 to 1300 mg per day, more preferably 80 to 1200 mg per day, most preferably 100 to 1100 mg per day.

In an embodiment when the composition is in the form of a tube feed the daily amount of choline or choline salt administered is preferably 200 to 1400 mg per day, more preferably 300 to 1200 mg per day, most preferably 350 to 1100 mg per day.

In another embodiment when the composition is in the form of a oral nutritional supplement the daily amount of choline or choline salt administered is preferably 70 to 500 mg per day, more preferably 80 to 400 mg per day, most preferably 100 to 300 mg per day.

The present composition preferably comprises 30 to 180 mg a) choline or choline salts per 100 ml of the liquid composition, preferably 35 mg to 170 mg choline per 100 ml, more preferably 40 to 160 mg choline per 100 ml composition, most preferably 45 mg to 150 mg choline per 100 ml.

Based on calories, preferably the nutrional compostion according to the invention comprises 25 to 110 mg choline per 100 kcal, preferably 30 to 105 mg choline per 100 kcal, more preferably 33 to 100 mg per 100 kcal based on the total energy content of the nutritional composition.

The above numbers are based on choline, the amounts of choline salts or esters can be calculated taking the molar equivalent to choline into account.

The present composition may comprise choline in the form of a choline ester, preferably provided in the form of phospholipids. Preferably, one or more phospholipid(s) is/are present in the composition according to the invention. The one or more phospholipid(s) is/are selected from the group consisting of phosphatidic acid (PA), phosphatidylethanolamine (PE), phosphatidylcholine (PC), phosphatidylserine (PS) and phosphoinositides (PI), preferably at least phosphatidyl choline. Phospholipids are in a preferred embodiment a source of the choline ester phosphatidylcholine.

Suitable sources of phospholipids herein are soya lecithin, egg lecithin as well as rapeseed and sunflower lecithin. Milk contains phospholipids in the milk fat globule membrane (MFGM), suitable sources include milk phospholipid concentrate such as those commercially available from Lecico GmbH, whey protein concentrate Lacprodan^{®} |MFGM from Arla Foods. Depending on the source, phospholipids comprise about 10 to 40 wt% of phosphatidylcholine.

If present, the composition and use of the composition preferably comprises the administration of phospholipids in an amount of 0.6 to 6.6 g mg per day, preferably 0.8 to 6.4 g of phospholipids per day, more preferably 0.9 to 6.2 g of phospholipids per day, most preferably 1 to 6 g of phospholipids per day. In an aspect the amount of phospholipids is about 200 mg per day. The phospholipids preferably at least provide phosphatidylcholine.

If phospholipids are present, the present composition preferably comprises phospholipids in an amount of 150 and 900 mg per 100 ml, more preferably between 200 and 850 mg per 100 ml, most preferably 250 to 800 mg per 100 ml. The phospholipids preferably at least provide phosphatidylcholine.

In an embodiment when the composition is in the form of a tube feed the daily amount of phospholipids administered is preferably 1 to 7 g per day, more preferably 1.5 to 6.5 g per day, most preferably 2 to 6 g per day.

In another embodiment when the composition is in the form of a oral nutritional supplement the daily amount of phospholipids administered is preferably 0.5 to 4 g per day, more preferably 0.75 to 3.5 g per day, most preferably 1 to 3 g per day.

Based on calories, preferably the nutrional compostion according to the invention comprises 100 to 500 mg of phospholipids per 100 kcal, preferably 150 to 400 mg of phospholipids per 100 kcal, more preferably 200 to 350 mg of phospholipids per 100 kcal based on the total energy content of the nutritional composition.

In a specific embodiment, the nutritional composition according to the invention, comprises a) choline or choline salts and b) phosphatidylcholine (PC). In such embodiment the weight to weight ratio phosphatidylcholine to choline is usually more than 0.1, preferably more than 0.26, in particular 0.30-6, more preferably 0.36-3. In a further preferred embodiment the ratio phosphatidylcholine to choline is in the range of 40:60 to 60:40. Herein, the amount of choline in grams is to be calculated as the molar contribution of choline as provided by all choline sources (when orally digested and assuming 100% bioavailability, including PC), times the molecular weight of choline (104 g/mol). Herein, the molecular weight of PC is 810 gram/mol.So for example, including 400 mg choline chloride (having a molecular weight of 139.6g/mol) and 200 mg phosphatidylcholine and 200 mg PL's other than PC would then result in a weight ratio of PC to choline of 200/ [(104/139.6)x400 + (104/810)x200] = 200/[298 + 25.7] = 0.62. The presence of PC is in particular preferred since herewith the choline (salt) content can be reduced, whilst still providing a bioavailable choline source. Additionally dietary choline can be metabolized in the gut into trimethylamine (TMA) which in turn is oxidized in the liver into trimethylamine N-oxide (TMAO). Both TMA and TMAO have been associated with an increased risk of cardiovascular diseases (Zhu et al, Cell 2016; Jaworska et al. Cardiovac. Res 2019) and therefore it is preferred to provide choline both in free (salt) form and as phosphatidylcholine.

Preferably the composition according to the invention comprising the combination of (i) coenzyme Q10, (ii) vitamin D, (iii) vitamin B2, (iv) polyunsaturated fatty acids and (v) choline further comprises one of more selected from A, further B, C, and E vitamin(s), phospholipids, selenium and magnesium. More preferred the composition contains two or more of these further ingredients. The further ingredients and their respective amounts will be discussed hereinafter.

### Vitamin A

The present composition may further comprise vitamin A. Vitamin A is a fat-soluble vitamin which comes in several forms (as retinol, retinal, retinoic acid or retinyl ester). Any functional form of vitamin A known in the art is suitable to be used herein, including retinol (in particular retinol esters), retinal, retinoic acid, beta-carotene, provitamin A, or any combination thereof. Preferably, the composition comprises retinol, in particular retinyl acetate and/or retinyl palmitate.

Vitamin A intakes or requirements are generally expressed in terms of retinol equivalents (RE). One RE is defined as the biological activity associated with 1 µg of all-trans retinol. Hence 1 µg Retinol Equivalent is similar to 1 µg of all-trans retinol. 1 International Unit (IU) retinol corresponds to 0.3 µg Retinol Equivalents.

If present in the composition, vitamin A is preferably administered in an amount of 300 to 2800 µg Retinol Equivalents per day, more preferably 350 to 2700 µg Retinol Equivalents per day, even more preferably 650 to 900 µg Retinol Equivalents per day, most preferably 375 to 2600 µg Retinol Equivalents per day.

In an embodiment the composition is in the form of a tube feed and the daily amount of vitamin A administered is preferably 700 to 2850 µg Retinol Equivalents per day, more preferably 800 to 2750 µg Retinol Equivalents per day, most preferably 850 to 2600 µg Retinol Equivalents per day.

In another embodiment the composition is in the form of a oral nutritional supplement and the daily amount of vitamin A administered is preferably 300 to 1200 µg Retinol Equivalents per day, more preferably 350 to 1150 µg Retinol Equivalents per day, most preferably 375 to 1125 µg Retinol Equivalents per day.

The present composition may preferably comprise vitamin A, preferably 50 to 400 µg Retinol Equivalents per 100 ml of the composition, more preferably 75 to 350 µg Retinol Equivalents per 100 ml, most preferably 100 to 300 µg Retinol Equivalents per 100 ml composition.

Based on calories, preferably the nutrional compostion according to the invention may comprise 60 to 150 µg Retinol Equivalents per 100 kcal, preferably 70 to 140 µg Retinol Equivalents per 100 kcal, more preferably 80 to 130 µg Retinol Equivalents per 100 kcal based on the total energy content of the nutritional composition.

### Vitamin C

The present composition may further comprise vitamin C. Vitamin C is an antioxidant that may provide beneficial characteristics during and after a period of hypoxia such as a stroke. Vitamin C includes functional equivalents thereof including sodium ascorbate and calcium ascorbate, and may be present in an amount to provide a daily dosage in the range of 75 to 700 mg, preferably in the range of 85 to 600 mg, more preferably in the range of 95 to 550 mg per day. When the composition is in the form of a tube feed the daily amount of vitamin C administered is preferably 140 to 600 mg per day, more preferably 160 to 575 mg per day, most preferably 180 to 550 mg per day. When the composition is in the form of a oral nutritional supplement the daily amount of vitamin C administered is preferably 80 to 320 mg per day, more preferably 90 to 300 mg per day, most preferably 95 to 290 mg per day. In one embodiment, if present vitamin C is present in an amount in the range of 10 to 100 mg, preferably in the range of 15 to 90 mg, more preferably in the range of 20 to 80 mg per 100 ml of the composition. Based on calories, preferably the compostion according to the invention may comprise 12 to 40 mg vitamin C per 100 kcal, preferably 14 to 35 mg vitamin C per 100 kcal, more preferably 16 to 32 mg vitamin C per 100 kcal based on the total energy content of the composition. The above numbers are based on the molar weight of ascorbic acid.

### Vitamin E

The composition according to the invention may comprise vitamin E. Vitamin E refers to compounds having vitamin E activity as known in the art, typically tocopherol and/or an equivalent thereof.

If included, vitamin E may be present in the composition in an amount to provide a daily dosage in the range of 8 to 75 mg, preferably in the range of 10 to 70 mg, more preferably in the range of 12.5 to 70 mg alpha-TE. When the composition is in the form of a tube feed the daily amount of vitamin E administered is preferably 18 to 80 mg per day, more preferably 20 to 75 mg per day, most preferably 22 to 70 mg alpha-TE per day. When the composition is in the form of a oral nutritional supplement the daily amount of vitamin E administered is preferably 8 to 42 mg per day, more preferably 10 to 40 mg per day, most preferably 12.5 to 37.5 mg alpha-TE per day. Such amounts of vitamin E prevent oxidative damage to the injury site. In one embodiment, tocopherol and/or equivalent is present in an amount in the range of 2 to 14 mg alpha-TE, preferably in the range of 2.5 to 12 mg alpha-TE, more preferably in the range of 2.75 to 10 mg alpha-TE per 100 ml of the composition. Based on calories, preferably the compostion according to the invention may comprise 1 to 5.5 mg alpha-TE per 100 kcal, preferably 1.5 to 5 mg alpha-TE per 100 kcal, more preferably 2 to 4.5 mg alpha-TE per 100 kcal based on the total energy content of the composition.

The term "tocopherol and/or an equivalent thereof", as used in this description, comprises tocopherols (e.g. alpha- and gamma-), tocotrienols, pharmaceutical and/or nutritional acceptable derivatives thereof and any combination thereof. The above numbers are based on alpha-tocopherol equivalents (alpha-TE) and the molecular weight thereof, as recognized in the art.

### Vitamin K1

The composition according to the invention may optionally comprise vitamin K, preferably vitamin K1, although it is preferred that the composition does not comprise or comprise minimal amounts of vitamin K. Vitamin K is a general name for a group of compounds containing a 2-methyl-1,4-naphthoquinone nucleus with different lipophilic side chains at position 3, in the following also referred to as "the K-vitamins". Vitamin K naturally occurs in the form of vitamin K1 (phytomenadione, phytonadione, α-phylloquinone) and vitamin K2 (menaquinone) which is produced by gastrointestinal bacteria in basically two different sub-types, namely vitamin K2(35) (C46H64O2) and vitamin K2(30) (C41H56O2). Vitamin K3 (menadione) is a pro-vitamin which can be converted to vitamin K2 by animals.

If present in the composition, the vitamin K1 is preferably present in a daily dosage at most 240 µg, preferably at most 200 µg, more preferably at most 100 µg per day. The present composition preferably comprises less than 40 µg vitamin K1 per 100 ml, more preferably less than 35 µg vitamin K1 per 100 ml, more preferably less than 30 µg vitamin K1 per 100 ml. Based on calories, preferably the compostion according to the invention may comprise less than 18 µg vitamin K1 per 100 kcal, preferably less than 16 µg vitamin K1 per 100 kcal, more preferably less than 15 µg vitamin K1 per 100 kcal based on the total energy content of the composition. The above numbers are based on the molar weight of phylloquinone.

In a preferred embodiment the composition comprises less than 15 µg vitamin K1 per 100 kcal.

### Selenium

The present composition may comprise selenium. The antioxidant activity of selenium may advantageously prevent and/or inhibit damages to brain areas.

If present in the composition, selenium is preferably present in an amount to provide a daily dosage in the range of 30 to 275 µg, preferably in the range of 35 to 250 µg, more preferably in the range of 38 to 225 µg per day. When the composition is in the form of a tube feed the daily amount of selenium administered is preferably 60 to 275 µg per day, more preferably 70 to 250 µg per day, most preferably 75 to 225 µg per day. When the composition is in the form of a oral nutritional supplement the daily amount of selenium administered is preferably 25 to 140 µg per day, more preferably 30 to 130 µg per day, most preferably 35 to 120 µg per day. The present composition preferably comprises 8 to 45 µg selenium per 100 ml, more preferably 10 to 40 µg selenium per 100 ml, more preferably 11 to 35 µg selenium per 100 ml. Based on calories, preferably the compostion according to the invention may comprise 4 to 18 µg selenium per 100 kcal, preferably 6 to 16 µg selenium per 100 kcal, more preferably 8 to 14 µg selenium per 100 kcal based on the total energy content of the composition.

### Magnesium

The present composition may comprise magnesium. Magnesium plays an essential role in nerve transmission and neuromuscular conduction. It also functions in a protective role against excessive excitation that can lead to neuronal cell death (excitotoxicity).

If present in the composition, magnesium is preferably present in an amount to provide a daily dosage in the range of 125 to 850 mg, preferably in the range of 150 to 800 mg, more preferably in the range of 170 to 750 mg per day. When the composition is in the form of a tube feed the daily amount of magnesium administered is preferably 200 to 780 mg per day, more preferably 220 to 760 mg per day, most preferably 240 to 740 mg per day. When the composition is in the form of a oral nutritional supplement the daily amount of magnesium administered is preferably 150 to 600 mg per day, more preferably 160 to 550 mg per day, most preferably 170 to 525 mg per day. The present composition preferably comprises 20 to 160 mg magnesium per 100 ml, more preferably 25 to 150 mg magnesium per 100 ml, more preferably 30 to 140 mg magnesium per 100 ml. Based on calories, preferably the compostion according to the invention may comprise 10 to 70 mg magnesium per 100 kcal, preferably 15 to 65 magnesium per 100 kcal, more preferably 20 to 60 mg magnesium per 100 kcal based on the total energy content of the composition.

The composition may comprise further minerals including but not limited to sodium, potassium, chloride, calcium, phosphor, iron, zinc, cupper, manganese, fluor, molybdenum, chrome and iodium. Amount thereof comply with general recommendations for nutritional requirements of the FSMP.

### LA/ALA

The lipid fraction may in addition to the LC-PUFAs further comprise lipids that are a source of essential fatty acids alpha-linolenic acid (ALA, C-18:3) and linoleic acid (LA, C-18:2). LA and/or ALA may be provided as free fatty acids, in triglyceride form, in diglyceride form, in monoglyceride form, in phospholipid form, or as a mixture of one of more of the above. Preferably the present composition contains at least one, preferably at least two lipid sources selected from the group consisting of soybean oil, rape seed oil (such as colza oil, low erucic acid rape seed oil and canola oil), high oleic sunflower oil, high oleic safflower oil and olive oil. The LA:ALA weight ratio is preferably in the range of 8:1 to 3:1, preferably 7:1 to 4:1, more preferably 6:1 to 5:1.

If present in the composition, the present composition preferably comprises 0.2 to 3.5 g LA per 100 ml, more preferably 0.3 to 3 g LA per 100 ml, more preferably 0.4 to 2.5 g LA per 100 ml. Based on calories, preferably the compostion according to the invention may comprise 0.1 to 1.5 g LA per 100 kcal, preferably 0.2 to 1.1 g LA per 100 kcal, more preferably 0.25 to 1 g LA per 100 kcal based on the total energy content of the composition.

If present in the composition, the present composition preferably comprises 0.02 to 0.2 g ALA per 100 ml, more preferably 0.05 to 0.15 g ALA per 100 ml, more preferably 0.06 to 0.1 g ALA per 100 ml. Based on calories, preferably the compostion according to the invention may comprise 0.03 to 0.08 g ALA per 100 kcal, preferably 0.04 to 0.07 g ALA per 100 kcal, more preferably 0.05 to 0.065 g ALA per 100 kcal based on the total energy content of the composition.

In an embodiment a composition is provided suitable for subjects that suffered from a stroke, preferably an ischemic stroke.

Such a nutritional composition according to the invention suitably comprises a combination of (i) co-enzyme Q10, (ii) vitamin D, (iii) vitamin B2, (iv) polyunsaturated fatty acids and (v) choline In an embodiment the composition further comprises one or more of B6, and B9. Preferably, the composition according to the invention further comprises one of more selected from A, further B, C and E vitamin(s), phospholipids, selenium and magnesium.

Preferably, the composition according to the invention further comprises one of more selected from A, B, C and E vitamin(s), phospholipids, selenium and magnesium. Preferably the composition according to the invention further comprises one or more of vitamin B1, B6, B9 and vitamin B12. In a further preferred embodiment, the composition comprises vitamin D3, co-enzyme Q10, vitamin B2, polyunsaturated fatty acids, choline and one or more, preferably all, of vitamin B1, B6, B9, B12, vitamin A, vitamin C, vitamin E, selenium, magnesium and phospholipids.

In an embodiment the composition according to the invention comprises per daily dosage,
(i) 8 to 80 mg, preferably 10 to 70 mg, more preferably 15 to 60 mg co-enzyme Q10; with
(ii) 6 to 75 µg, preferably 8 to 70 µg, more preferably 10 to 65 µg vitamin D, preferably vitamin D3; and
(iii) 0.8 to 7.6 mg, preferably 1 to 7.4 mg, more preferably in the range of 1.2 to 7.2 mg vitamin B2; and optionally
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA; and/or
(v) 200 to 1400 mg, preferably 300 to 1200 mg, more preferably 350 to 1100 mg choline or choline salts.

In an embodiment the composition according to the invention comprises per daily dosage,
(i) 8 to 80 mg, preferably 10 to 70 mg, more preferably 15 to 60 mg co-enzyme Q10; with
(ii) 6 to 75 µg, preferably 8 to 70 µg, more preferably 10 to 65 µg vitamin D, preferably vitamin D3; and
(iii) 0.8 to 7.6 mg, preferably 1 to 7.4 mg, more preferably in the range of 1.2 to 7.2 mg vitamin B2;
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA; and
(v) 200 to 1400 mg, preferably 300 to 1200 mg, more preferably 350 to 1100 mg choline based on molar weight of choline, wherein choline is selected from free choline, choline salts and/or choline esters, preferably at least phosphatidylcholine.

In an embodiment the composition according to the invention comprises per daily dosage,
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3; and optionally
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA;
(v) 200 to 1400 mg, preferably 300 to 1200 mg, more preferably 350 to 1100 mg choline or choline salts.

In a further embodiment the composition according to the invention comprises per daily dosage:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3;
(iii) 0.8 to 7.6 mg, preferably 1 to 7.4 mg, more preferably 1.2 to 7.2 mg vitamin B2; in combination with at least one (vi) and (vii)
(vi) 0.7 to 9 mg, preferably 0.8 to 8 mg, more preferably 0.9 to 7.5 mg vitamin B6;
(vii) 150 to 900 µg, preferably 175 to 800 µg, more preferably 200 to 750 µg vitamin B9 and optionally
(iv) 150 to 1200 mg, preferably 200 to 1100 mg, more preferably 250 to 1000 mg DHA; and/or 300 to 2400 mg, preferably 400 to 2200 mg, more preferably 500 to 2000 mg per day EPA;
(v) 200 to 1400 mg, preferably 300 to 1200 mg, more preferably 350 to 1100 mg choline or choline salts.

In a further embodiment the composition according to the invention comprises per daily dosage:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3;
(iii) 0.8 to 7.6 mg, preferably 1 to 7.4 mg, more preferably 1.2 to 7.2 mg vitamin B2;
   in combination with
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA;
(v) 200 to 1400 mg, preferably 300 to 1200 mg, more preferably 350 to 1100 mg choline, wherein choline is selected from free choline, choline salts and/or choline esters, preferably at least phosphatidylcholine.

In a further embodiment the composition according to the invention comprises per daily dosage:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3;
(iii) 0.8 to 7.6 mg, preferably 1 to 7.4 mg, more preferably 1.2 to 7.2 mg vitamin B2;
   in combination with
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA;
(v) 200 to 1400 mg, preferably 300 to 1200 mg, more preferably 350 to 1100 mg choline, wherein choline is selected from free choline, choline salts and/or choline esters, preferably at least phosphatidylcholine.

In a further embodiment the composition according to the invention comprises per daily dosage:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3;
(iii) 0.8 to 7.6 mg, preferably 1 to 7.4 mg, more preferably 1.2 to 7.2 mg vitamin B2;
   in combination with
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA;
(v) 200 to 1400 mg, preferably 300 to 1200 mg, more preferably 350 to 1100 mg choline, wherein choline is selected from free choline, choline salts and/or choline esters, wherein the composition preferably comprises vₐ) phosphatidylcholine and v_{b}) choline or a salt thereof, and wherein the weight to weight ratio of phosphatidylcholine to choline is more than 0.1.

In a further embodiment the composition according to the invention comprises per daily dosage:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3;
(iii) 0.8 to 7.6 mg, preferably 1 to 7.4 mg, more preferably 1.2 to 7.2 mg vitamin B2;
   in combination with
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA;
(v) 200 to 1400 mg, preferably 300 to 1200 mg, more preferably 350 to 1100 mg choline, wherein choline is selected from free choline, choline salts and/or choline esters, preferably at least phosphatidylcholine.

In a further embodiment the composition according to the invention comprises per daily dosage:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg vitamin D, preferably vitamin D3;
(iii) 0.8 to 7.6 mg, preferably 1 to 7.4 mg, more preferably 1.2 to 7.2 mg vitamin B2;
   in combination with
(iv) 150 to 1200 mg, preferably 200 to 1100 mg, more preferably 250 to 1000 mg DHA; and/or
   300 to 2400 mg, preferably 400 to 2200 mg, more preferably 500 to 2000 mg per day EPA;
(v) 200 to 1400 mg, preferably 300 to 1200 mg, more preferably 350 to 1100 mg choline, wherein choline is selected from free choline, choline salts and/or choline esters, wherein the composition preferably comprises vₐ) phosphatidylcholine and v_{b}) choline or a salt thereof, and wherein the weight to weight ratio of phosphatidylcholine to choline is more than 0.1.

In a further preferred embodiment the composition comprising per daily dosage a combination of (i) (ii) and (iii), at least one of (vi) and (vii) and optionally (iv) and/or (v) further comprises one or more of
(viii) 350 to 2700 µg Retinol Equivalents, preferably 650 to 900 µg Retinol Equivalents, more preferably 375 to 2600 µg Retinol Equivalents of vitamin A;
(ix) 0.7 to 5 mg, preferably 0.8 to 4.9 mg, more preferably 0.9 to 4.8 mg vitamin B1;
(x) 2 to 20 µg, preferably 2.5 to 19 µg, more preferably 3 to 18 µg vitamin B12;
(xi) 75 to 700 mg, preferably 85 to 600 mg, more preferably 95 to 550 mg vitamin C;
(xii) 8 to 75 mg, preferably 10 to 70 mg, more preferably 12.5 to 70 mg alpha-TE;
(xiii) 30 to 275 µg, preferably 35 to 250 µg, more preferably 38 to 225 µg selenium; and,
(xiv) 125 to 850 mg, preferably 150 to 800 mg, more preferably 170 to 750 mg magnesium.

In a further preferred embodiment the composition comprising per daily dosage a combination of (i) (ii), (iii),(iv) and (v) at least one of (vi) and (vii) and one or more of
(viii) 350 to 2700 µg Retinol Equivalents, preferably 650 to 900 µg Retinol Equivalents, more preferably 375 to 2600 µg Retinol Equivalents of vitamin A;
(ix) 0.7 to 5 mg, preferably 0.8 to 4.9 mg, more preferably 0.9 to 4.8 mg vitamin B1;
(x) 2 to 20 µg, preferably 2.5 to 19 µg, more preferably 3 to 18 µg vitamin B12;
(xi) 75 to 700 mg, preferably 85 to 600 mg, more preferably 95 to 550 mg vitamin C;
(xii) 8 to 75 mg, preferably 10 to 70 mg, more preferably 12.5 to 70 mg alpha-TE;
(xiii) 30 to 275 µg, preferably 35 to 250 µg, more preferably 38 to 225 µg selenium; and,
(xiv) 125 to 850 mg, preferably 150 to 800 mg, more preferably 170 to 750 mg magnesium.

In a further preferred embodiment the composition comprises per daily dosage a combination of (i) (ii) and (iii), at least one of (vi) and (vii) and optionally (iv) and/or (v), one or more selected of (vii) - (xiv) further comprises at least one of
(xv) 3 to 45 mg, preferably 3.5 to 42 mg, more preferably 3.8 to 40 mg vitamin B3 (niacin equivalents);
(xvi) 0.8 to 15 mg, preferably 1 to 14 mg, more preferably 1.2 to 13 mg vitamin B5;
(xvii) 8 to 95 µg, preferably in the range of 9 to 90 µg, more preferably in the range of 9.5 to 85 µg vitamin B7.

Provided herein is also a liquid nutritional composition comprising per 100 ml
(i) 1 to 15 mg, preferably 1.5 to 14 mg, more preferably 2 to 13 mg co-enzyme Q10; with
(ii) 1.5 to 10 µg, preferably 2 to 9 µg, more preferably 2.5 to 8 µg vitamin D, preferably vitamin D3; and
(iii) 0.1 to 1.4 mg, preferably 0.2 to 1.2 mg, more preferably 0.3 to 1 mg vitamin B2; and optionally
(iv) 30 to 240 mg, preferably 35 to 220 mg, more preferably 40 to 200 mg DHA; and/or
   70 to 440 mg, preferably 80 to 420 mg, more preferably 90 to 400 mg EPA; and/or,
(v) 35 to 170 mg, preferably 40 to 160 mg, more preferably 45 to 150 mg choline or choline salts.

Provided herein is also a liquid nutritional composition comprising per 100 ml
(i) 1 to 15 mg, preferably 1.5 to 14 mg, more preferably 2 to 13 mg co-enzyme Q10; with
(ii) 1.5 to 10 µg, preferably 2 to 9 µg, more preferably 2.5 to 8 µg vitamin D, preferably vitamin D3; and
(iii) 0.1 to 1.4 mg, preferably 0.2 to 1.2 mg, more preferably 0.3 to 1 mg vitamin B2; and optionally
(iv) 30 to 240 mg, preferably 35 to 220 mg, more preferably 40 to 200 mg DHA; and/or
   70 to 440 mg, preferably 80 to 420 mg, more preferably 90 to 400 mg EPA; and/or,
(v) 35 to 170 mg, preferably 40 to 160 mg, more preferably 45 to 150 mg choline, wherein choline is selected from free choline, choline salts and/or choline esters, preferably at least phosphatidylcholine.

Provided herein is also a liquid nutritional composition comprising per 100 ml
(i) 1 to 15 mg, preferably 1.5 to 14 mg, more preferably 2 to 13 mg co-enzyme Q10;
(ii) 1.5 to 10 µg, preferably 2 to 9 µg, more preferably 2.5 to 8 µg vitamin D, preferably vitamin D3;
(iii) 0.1 to 1.4 mg, preferably 0.2 to 1.2 mg, more preferably 0.3 to 1 mg vitamin B2;
(iv) 30 to 240 mg, preferably 35 to 220 mg, more preferably 40 to 200 mg DHA; and/or
   70 to 440 mg, preferably 80 to 420 mg, more preferably 90 to 400 mg EPA;
   and
(v) 35 to 170 mg, preferably 40 to 160 mg, more preferably 45 to 150 mg choline.

Provided herein is also a liquid nutritional composition comprising per 100 ml
(i) 1 to 15 mg, preferably 1.5 to 14 mg, more preferably 2 to 13 mg co-enzyme Q10;
(ii) 1.5 to 10 µg, preferably 2 to 9 µg, more preferably 2.5 to 8 µg vitamin D, preferably vitamin D3;
(iii) 0.1 to 1.4 mg, preferably 0.2 to 1.2 mg, more preferably 0.3 to 1 mg vitamin B2;
(iv) 30 to 240 mg, preferably 35 to 220 mg, more preferably 40 to 200 mg DHA; and/or
   70 to 440 mg, preferably 80 to 420 mg, more preferably 90 to 400 mg EPA;
(v) 35 to 170 mg, preferably 40 to 160 mg, more preferably 45 to 150 mg choline, wherein choline is selected from free choline, choline salts and/or choline esters, preferably at least phosphatidylcholine.

Further provided herein is a liquid nutritional composition comprising per 100 ml
(i) 1 to 15 mg, preferably 1.5 to 14 mg, more preferably 2 to 13 mg co-enzyme Q10; with
(ii) 1.5 to 10 µg, preferably 2 to 9 µg, more preferably 2.5 to 8 µg vitamin D, preferably vitamin D3;
   and
(iii) 0.1 to 1.4 mg, preferably 0.2 to 1.2 mg, more preferably 0.3 to 1 mg vitamin B2; and optionally
(iv) 30 to 240 mg, preferably 35 to 220 mg, more preferably 40 to 200 mg DHA; and/or
   70 to 440 mg, preferably 80 to 420 mg, more preferably 90 to 400 mg EPA; and/or,
(v) 35 to 170 mg, preferably 40 to 160 mg, more preferably 45 to 150 mg choline, wherein said choline is selected from free choline, choline salts and/or choline esters, preferably at least phosphatidylcholine and, wherein the composition preferably comprises vₐ) phosphatidylcholine and v_{b}) choline or a salt thereof, and wherein the weight to weight ratio of phosphatidylcholine to choline is more than 0.1.

In an further embodiment a liquid nutritional composition is provided comprising per 100 ml,
(i) 1 to 15 mg, preferably 1.5 to 14 mg, more preferably 2 to 13 mg co-enzyme Q10; with
(ii) 1.5 to 10 µg, preferably 2 to 9 µg, more preferably 2.5 to 8 µg vitamin D, preferably vitamin D3; and optionally
(iv) 30 to 240 mg, preferably 35 to 220 mg, more preferably 40 to 200 mg DHA; and/or
   70 to 440 mg, preferably 80 to 420 mg, more preferably 90 to 400 mg EPA; and/or,
(v) 35 to 170 mg, preferably 40 to 160 mg, more preferably 45 to 150 mg choline or choline salts.

In an further embodiment a liquid nutritional composition is provided comprising per 100 ml:
i) 1 to 15 mg, preferably 1.5 to 14 mg, more preferably 2 to 13 mg co-enzyme Q10; with
(ii) 1.5 to 10 µg, preferably 2 to 9 µg, more preferably 2.5 to 8 µg vitamin D, preferably vitamin D3;
(iii) 0.1 to 1.4 mg, preferably 0.2 to 1.2 mg, more preferably 0.3 to 1 mg vitamin B2;
   in combination with at least one of (vi) and (vii);
(vi) 0.2 to 1 mg, preferably 0.25 to 0.8 mg, more preferably 0.3 to 0.7 mg vitamin B6;
(vii) 20 to 225 µg, preferably 25 to 200 µg, more preferably 30 to 175 µg vitamin B9; and optionally
(iv) 30 to 240 mg, preferably 35 to 220 mg, more preferably 40 to 200 mg DHA; and/or
   70 to 440 mg, preferably 80 to 420 mg, more preferably 90 to 400 mg EPA; and/or,
(v) 35 to 170 mg, preferably 40 to 160 mg, more preferably 45 to 150 mg choline or choline salts.

In an further embodiment a liquid nutritional composition is provided comprising per 100 ml:
i) 1 to 15 mg, preferably 1.5 to 14 mg, more preferably 2 to 13 mg co-enzyme Q10;
(ii) 1.5 to 10 µg, preferably 2 to 9 µg, more preferably 2.5 to 8 µg vitamin D, preferably vitamin D3;
(iii) 0.1 to 1.4 mg, preferably 0.2 to 1.2 mg, more preferably 0.3 to 1 mg vitamin B2;
(iv) 30 to 240 mg, preferably 35 to 220 mg, more preferably 40 to 200 mg DHA; and/or
   70 to 440 mg, preferably 80 to 420 mg, more preferably 90 to 400 mg EPA;
(v) 35 to 170 mg, preferably 40 to 160 mg, more preferably 45 to 150 mg choline, and
   at least one of (vi) and (vii);
(vi) 0.2 to 1 mg, preferably 0.25 to 0.8 mg, more preferably 0.3 to 0.7 mg vitamin B6;
(vii) 20 to 225 µg, preferably 25 to 200 µg, more preferably 30 to 175 µg vitamin B9.

In an further embodiment a liquid nutritional composition is provided comprising per 100 ml:
i) 1 to 15 mg, preferably 1.5 to 14 mg, more preferably 2 to 13 mg co-enzyme Q10;
(ii) 1.5 to 10 µg, preferably 2 to 9 µg, more preferably 2.5 to 8 µg vitamin D, preferably vitamin D3;
(iii) 0.1 to 1.4 mg, preferably 0.2 to 1.2 mg, more preferably 0.3 to 1 mg vitamin B2;
(iv) 30 to 240 mg, preferably 35 to 220 mg, more preferably 40 to 200 mg DHA; and/or
   70 to 440 mg, preferably 80 to 420 mg, more preferably 90 to 400 mg EPA;
(v) 35 to 170 mg, preferably 40 to 160 mg, more preferably 45 to 150 mg choline, wherein said choline is selected from free choline, choline salts and/or choline esters, preferably at least phosphatidylcholine and, wherein the composition preferably comprises vₐ) phosphatidylcholine and v_{b}) choline or a salt thereof, and wherein the weight to weight ratio of phosphatidylcholine to choline is more than 0.1;
   and at least one of (vi) and (vii);
(vi) 0.2 to 1 mg, preferably 0.25 to 0.8 mg, more preferably 0.3 to 0.7 mg vitamin B6;
(vii) 20 to 225 µg, preferably 25 to 200 µg, more preferably 30 to 175 µg vitamin B9.

In a further preferred embodiment the liquid nutritional composition comprising per 100 ml a combination of (i) and (ii), (iii), at least one of (vi) and (vii) and optionally (iv) and/or to (v), further comprises one or more of
(viii) 50 to 400 µg, preferably 75 to 350 µg, more preferably 100 to 300 µg Retinol Equivalents of vitamin A;
(ix) 0.1 to 0.9 mg, preferably 0.15 to 0.8 mg, more preferably 0.2 to 0.75 mg vitamin B1;
(x) 0.5 to 3 µg, preferably 0.6 to 2.6 µg, more preferably 0.7 to 2.4 µg vitamin B12;
(xi) 10 to 100 mg, preferably 15 to 90 mg, more preferably 20 to 80 mg vitamin C;
(xii) 2 to 14 mg, preferably 2.5 to 12 mg, more preferably 2.75 to 10 mg alpha-TE;
(xiii) 8 to 45 µg, preferably 10 to 40 µg, more preferably 11 to 35 µg selenium; and,
(xiv) 20 to 160 mg, preferably 25 to 150 mg, more preferably 30 to 140 mg magnesium.

In a further preferred embodiment the liquid nutritional composition may further comprise per 100 ml one of more of
(xv) 1.2 to 6 mg, preferably 1.4 to 5.5 mg, more preferably 1.6 to 5 mg vitamin B3 (niacin equivalents);
(xvi) 0.3 to 2.2 mg. preferably 0.4 to 2 mg, more preferably 0.5 to 1.8 mg vitamin B5;
(xvii) 2 to 14 mg, preferably 3 to 12 mg, more preferably 3.5 to 11 mg vitamin B7;

Provided herein is further a liquid nutritional composition comprising per 100 kcal
(i) 1.5 to 5.5 mg, preferably 1.8 to 5.2 mg, more preferably 2 to 5 mg co-enzyme Q10;
(ii) 1.3 to 4.5 ug, preferably 1.5 to 4 ug, more preferably 2 to 3.5 µg vitamin D, preferably vitamin D3;
(iii) 0.4 to 0.6 mg, preferably 0.15 to 0.5 mg, more preferably 0.2 to 0.4 mg vitamin B2;
   and optionally
(iv) 20 to 100 mg, preferably 25 to 90 mg, more preferably 30 to 80 mg DHA; and/or
   20 to 225 mg, preferably 60 to 200 mg, more preferably 70 to 170 mg EPA; and/or
(v) 100 to 1000 mg, preferably 200 to 800 mg, more preferably 300 to 500 mg choline or choline salts.

Provided herein is also a liquid nutritional composition comprising per 100 kcal
(i) 1.5 to 5.5 mg, preferably 1.8 to 5.2 mg, more preferably 2 to 5 mg co-enzyme Q10;
(ii) 1.3 to 4.5 ug, preferably 1.5 to 4 ug, more preferably 2 to 3.5 µg vitamin D, preferably vitamin D3;
(iii) 0.4 to 0.6 mg, preferably 0.15 to 0.5 mg, more preferably 0.2 to 0.4 mg vitamin B2;
   and optionally
(iv) 20 to 100 mg, preferably 25 to 90 mg, more preferably 30 to 80 mg DHA; and/or
   20 to 225 mg, preferably 60 to 200 mg, more preferably 70 to 170 mg EPA; and/or
(v) 100 to 1000 mg, preferably 200 to 800 mg, more preferably 300 to 500 mg choline, wherein said choline is selected from free choline, choline salts and/or choline esters, preferably at least phosphatidylcholine and, wherein the composition preferably comprises vₐ) phosphatidylcholine and v_{b}) choline or a salt thereof, and wherein the weight to weight ratio of phosphatidylcholine to choline is more than 0.1.

In an embodiment a liquid nutritional composition is provided comprising per 100 kcal
(i) 1.5 to 5.5 mg, preferably 1.8 to 5.2 mg, more preferably 2 to 5 mg co-enzyme Q10;
(ii) 1.3 to 4.5 ug, preferably 1.5 to 4 ug, more preferably 2 to 3.5 µg vitamin D, preferably vitamin D3;
(iii) 0.4 to 0.6 mg, preferably 0.15 to 0.5 mg, more preferably 0.2 to 0.4 mg vitamin B2;
(iv) 20 to 100 mg, preferably 25 to 90 mg, more preferably 30 to 80 mg DHA; and/or
   20 to 225 mg, preferably 60 to 200 mg, more preferably 70 to 170 mg EPA;
(v) 100 to 1000 mg, preferably 200 to 800 mg, more preferably 300 to 500 mg choline.

In an embodiment there is also provided a liquid nutritional composition comprising per 100 kcal
(i) 1.5 to 5.5 mg, preferably 1.8 to 5.2 mg, more preferably 2 to 5 mg co-enzyme Q10;
(ii) 1.3 to 4.5 ug, preferably 1.5 to 4 ug, more preferably 2 to 3.5 µg vitamin D, preferably vitamin D3;
(iii) 0.4 to 0.6 mg, preferably 0.15 to 0.5 mg, more preferably 0.2 to 0.4 mg vitamin B2;
(iv) 20 to 100 mg, preferably 25 to 90 mg, more preferably 30 to 80 mg DHA; and/or
   20 to 225 mg, preferably 60 to 200 mg, more preferably 70 to 170 mg EPA;
(v) 100 to 1000 mg, preferably 200 to 800 mg, more preferably 300 to 500 mg choline, wherein said choline is selected from free choline, choline salts and/or choline esters, preferably at least phosphatidylcholine and, wherein the composition preferably comprises vₐ) phosphatidylcholine and v_{b}) choline or a salt thereof, and wherein the weight to weight ratio of phosphatidylcholine to choline is more than 0.1.

In an embodiment the liquid nutritional composition comprises per 100 kcal
(i) 1.5 to 5.5 mg, preferably 1.8 to 5.2 mg, more preferably 2 to 5 mg co-enzyme Q10;
(ii) 1.3 to 4.5 ug, preferably 1.5 to 4 ug, more preferably 2 to 3.5 µg vitamin D, preferably vitamin D3;
   and optionally
(iv) 20 to 100 mg, preferably 25 to 90 mg, more preferably 30 to 80 mg DHA; and/or
   20 to 225 mg, preferably 60 to 200 mg, more preferably 70 to 170 mg EPA; and/or
(v) 100 to 1000 mg, preferably 200 to 800 mg, more preferably 300 to 500 mg choline or choline salts.

In an further embodiment a liquid nutritional composition is provided comprising per 100 kcal:
(i) 1.5 to 5.5 mg, preferably 1.8 to 5.2 mg, more preferably 2 to 5 mg co-enzyme Q10;
(ii) 1.3 to 4.5 ug, preferably 1.5 to 4 ug, more preferably 2 to 3.5 µg vitamin D, preferably vitamin D3;
(iii) 0.4 to 0.6 mg, preferably 0.15 to 0.5 mg, more preferably 0.2 to 0.4 mg vitamin B2;in combination with at least one of (vi) and (vii);
(vi) 0.1 to 0.5 mg, preferably 0.2 to 0.4 mg, more preferably 0.25 to 0.3 mg vitamin B6;
(vii) 16 to 80 µg, preferably 18 to 75 µg, more preferably 20 to 70 µg vitamin B9;
   and optionally
(iv) 20 to 100 mg, preferably 25 to 90 mg, more preferably 30 to 80 mg DHA; and/or 20 to 225 mg, preferably 60 to 200 mg, more preferably 70 to 170 mg EPA; and/or
(v) 100 to 1000 mg, preferably 200 to 800 mg, more preferably 300 to 500 mg choline,choline salts and/or choline esters.

In a further preferred embodiment the liquid nutritional composition comprises per 100 kcal a combination of (i) (ii) and (iii), at least one of (vi) and (vii) and optionally (iv) and/or to (v), further comprises one or more of
(viii) 60 to 150 µg, preferably 70 to 140 µg, more preferably 80 to 130 µg Retinol Equivalents of vitamin A;
(ix) 0.05 to 0.4 mg, preferably 0.1 to 0.35 mg, more preferably 0.15 to 0.30 mg vitamin B1;
(x) 0.4 to 1.4 µg, preferably 0.5 to 1.2 µg, more preferably 0.6 to 1 µg vitamin B12;
(xi) 12 to 40 mg, preferably 14 to 35 mg, more preferably 16 to 32 mg vitamin C;
(xii) 1 to 5.5 mg, preferably 1.5 to 5 mg, more preferably 2 to 4.5 mg alpha-TE;
(xiii) 4 to 18 µg, preferably 6 to 16 µg, more preferably 8 to 14 µg selenium; and,
(xiv) 10 to 70 mg, preferably 15 to 65 mg, more preferably 20 to 60 mg magnesium.

In a further preferred embodiment the liquid nutritional composition may further comprise per 100 kcal one of more of
(xv) 1 to 2.5 mg, preferably 1.1 to 2.2 mg, more preferably 1.2 to 2 mg vitamin B3 (niacin equivalents);
(xvi) 0.2 to 1 mg. prefrably 0.3 to 0.9 mg, more preferably 0.4 to 0.8 mg vitamin B5;
(xvii) 1.5 to 6 mg, preferably 2 to 5.5 mg, more preferably 2.5 to 5 mg vitamin B7;

In a particular embodiment a composition is provided comprising
(i) co-enzyme Q10;
(ii) vitamin D, preferably vitamin D3;
(iii) vitamin B2;
(iv) DHA and EPA;
(v) choline or choline salts;
(vi) vitamin B6;
(vii) vitamin B9;
   and at least one or more of the following ingredients
(viii) Retinol Equivalents of vitamin A;
(ix) vitamin B1;
(x) vitamin B12;
(xi) vitamin C;
(xii) alpha-TE;
(xiii) selenium; and,
(xiv) magnesium.

In a further particular embodiment a composition is provided comprising
(i) co-enzyme Q10;
(ii) vitamin D, preferably vitamin D3;
(iii) vitamin B2;
(iv) DHA and EPA;
(v) choline, choline salts and/or choline ester, preferably at least phosphatidyl choline;
(vi) vitamin B6;
(vii) vitamin B9;
   and at least one or more of the following ingredients
(viii) Retinol Equivalents of vitamin A;
(ix) vitamin B1;
(x) vitamin B12;
(xi) vitamin C;
(xii) alpha-TE;
(xiii) selenium; and,
(xiv) magnesium.

### Examples

### Example 1

An exemplary enteral tube feed was formulated comprising per 100 ml 128 kcal and comprising per 100 kcal 4.88 g protein, 4.5 g lipids and 9.3 g carbohydrates. The tube feed comprises ingredients according to table 1 and is for administration to a tube to a patient after an ischemic stroke.

| Unit | Nutrient | Per 100 kcal |
|---|---|---|
| µg | Vitamin D3 | 2.08 |
| mg | Co-enzyme Q10 | 2 |
| g | DHA | 0.03 |
| g | EPA | 0.07 |
| mg | Choline | 36.67 |
| mg | Magnesium | 24.7 |
| µg | Selenium | 7.5 |
| µg RE | Vitamin A | 85.71 |
| mg α-TE | Vitamin E | 2.32 |
| mg | Thiamin | 0.16 |
| mg | Riboflavin | 0.24 |
| mg NE | Niacin (equivalent) | 1.29 |
| mg | Pantothenic acid | 0.42 |
| mg | Vitamin B6 | 0.25 |
| µg | Folic acid | 24.24 |
| µg | Vitamin B12 | 0.60 |
| µg | Biotin | 2.91 |
| mg | Vitamin C | 18.17 |
| g | Phospholipids | 0.20 |

### Example 2

An exemplary oral nutritional supplement was formulated comprising per 100 ml 240 kcal and comprising per 100 kcal 6 g protein, 3.89 g lipids and 9.57 g carbohydrates. The nutritional supplement comprises ingredients according to table 2 and is for administration as a drink to patients after an ischemic stroke.

| Unit | Nutrient | Per 100 kcal |
|---|---|---|
| µg | Vitamin D3 | 3.33 |
| mg | Co-enzyme Q10 | 5 |
| g | DHA | 0.08 |
| g | EPA | 0.17 |
| mg | Choline | 33.33 |
| mg | Magnesium | 58.3 |
| µg | Selenium | 13.3 |
| µg RE | Vitamin A | 125 |
| mg α-TE | Vitamin E | 4.17 |
| mg | Thiamin | 0.3 |
| mg | Riboflavin | 0.4 |
| mg NE | Niacin (equivalent) | 1.29 |
| mg | Pantothenic acid | 0.42 |
| mg | Vitamin B6 | 0.30 |
| µg | Folic acid | 66.67 |
| µg | Vitamin B12 | 1 |
| µg | Biotin | 3.5 |
| mg | Vitamin C | 31.67 |
| g | Phospholipids | 0.33 |

### Example 3 - Neurite outgrowth with co-enzyme Q10, vitamin D3 and vitamin B2

### NEURORESTORATIVE EFFECT OF NUTRIENT SUPPLEMENTATION AFTER HYPOXIA.

### Cortical neuronal cultures

Mouse primary cortical neurons (E14) were seeded onto 96-well plates coated with poly-D-lysine. Cells (20.000 cells/well) were cultured in modified Neurobasal medium (containing B27, Pen/Strep, and GlutaMAX). Cell cultures were incubated at 37 °C in 5% CO2 and medium was replaced every 2-3 days.

### Hypoxia: In vitro oxygen glucose deprivation

At day 2 of *in vitro* culturing, cells underwent oxygen glucose deprivation (OGD) for 3.5 hrs. In order to induce glucose deprivation, modified Neurobasal medium was replaced with an equivalent volume of HBSS. For hypoxia, cells were placed in a humidity-controlled incubator (Whitley H35 Hypoxystation) set to 0.5% O₂, 94.5% N₂ and 5% CO₂ at 37 °C for 3.5 hrs. After the hypoxic incubation period, HBSS was removed and replaced with modified Neurobasal medium, and plates returned to normoxic conditions and incubated at 37 °C in 5% CO₂ for an additional 24 hrs.

### Nutrient supplementation

The effects of nutrient supplementation of neuronal cultures after hypoxia were assessed by comparing different concentrations of vitamin D3 (30 nM), co-enzyme Q10 (3 µM), or vitamin B2 (30 nM) to the vehicle control (0 µM). Nutrients were supplemented either alone, in combination of two, or in combination of three, starting immediately after the OGD and lasting for another 24 hrs. Every condition was tested in triplicate. Control cells were exposed to hypoxia but not supplemented with any of vitamin D3, B2 or co-enzyme Q10.

### Neurite outgrowth: Immunohistochemistry and imaging

At the end of the experiment, plates were fixed PFA for 5 min at room temperature. Then, cells were permeabilized and blocked using PBS containing 10% normal goat serum, 0.1% sodium azide and 0.2%Triton X-100. Cells were stained overnight with a monoclonal anti-beta-tubulin III antibody (Sigma Aldrich, T8578). Next, cells were stained with the secondary antibody Alexa Fluor 488 Goat anti-Mouse IgG (H+L) (Invitrogen A11001 Fisher, 10256302). Nuclei were stained with Hoechst 33342 (Thermofisher, H3570). Images were taken at a magnification of 20x in 12 fields of view using In Cell Analyser 2000. Quantification of neurite outgrowth was performed using the InCell Developer Toolbox v1.6 software (GE Healthcare).

### Results

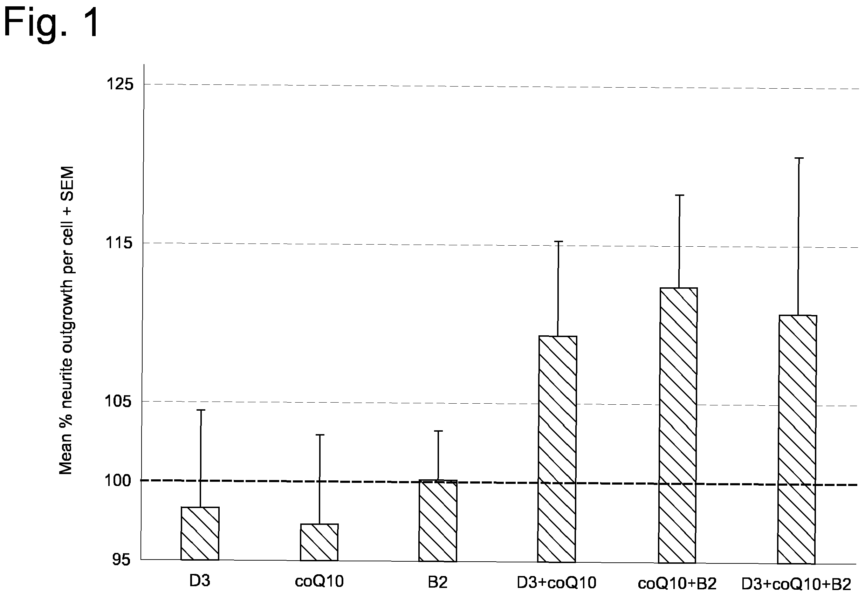

Image analysis of the neurite outgrowth after hypoxia showed that supplementation of vitamin D3, vitamin B2 or co-enzyme Q10 alone, did not affect neurite outgrowth as compared to control. However, when vitamin D3 and co-enzyme Q10 were combined, an increased neurite outgrowth was observed relative to control (set at 100%). A similar increase in neurite outgrowth relative to control was observed when vitamin B2 and co-enzymeQ10 were combined. These findings are depicted in Figure1.

### Example 4 - Neurite outgrowth with co-enzyme Q10, vitamin D3, vitamin B6 and B9

### NEURORESTORATIVE EFFECT OF NUTRIENT SUPPLEMENTATION AFTER HYPOXIA.

### Cortical neuronal cultures

Rat primary cortical neurons (E18) were seeded onto 96-well plates coated with poly-D-lysine. Cells (20.000 cells/well) were cultured in Neurobasal medium (containing B27, Pen/Strep, and GlutaMAX). Cell cultures were incubated at 37 °C in 5% CO2 and medium was replaced every 3-4 days.

### Hypoxia: In vitro oxygen glucose deprivation

At day 7 of in vitro culturing, cells underwent oxygen glucose deprivation (OGD) for 3.5 hrs. In order to induce glucose deprivation, modified Neurobasal medium was replaced with neurobasal medium without glucose. For hypoxia, cells were placed in a Hypoxia Incubator Chamber (Stemcell Technologies) set to 0.1% O2, 94.9% N2 and 5% CO2 at 37 °C for 3.5 hrs. After the hypoxic incubation period, medium was removed and replaced with modified Neurobasal medium, and plates returned to normoxic conditions and incubated at 37 °C in 5% CO2 for an additional 24 hrs.

### Nutrient supplementation

The effects of nutrient supplementation of neuronal cultures after hypoxia were assessed by comparing different concentrations of vitamin D (150 nM), co-enzyme Q10 (15 µM), vitamin B6 (1.5 µM), or vitamin B9 (2.25 µM) to the vehicle control (0 µM). Nutrients were supplemented either alone, in combination of two, or in combination of three, starting 24h after the OGD and lasting for two days. Every condition was tested in 5-fold. Control cells were exposed to hypoxia but not supplemented with any of vitamin D, B6, B9 or co-enzyme Q10.

### Neurite outgrowth: Immunohistochemistry and imaging

At the end of the experiment, plates were fixed with 4% PFA for 15 min at room temperature. Then, cells were permeabilized and blocked using an antibody buffer containing 20% normal goat serum, and 0.2% Triton X-100. Cells were stained overnight with a polyclonal anti-MAP2 antibody (Novusbio, NB300-213). Next, cells were stained with the secondary antibody CF-4A88 Goat anti-Chicken IgY (H+L) (Sigma, SAB4600039). Nuclei were stained with Hoechst 33342 (Thermo Scientific, #62249). Images were taken at a magnification of 10x in 9 fields of view using the ImageExpressMicro Confocal (Molecular Devices). Quantification of neurite outgrowth was performed using the MetaExpress software (Molecular Devices).

### Results

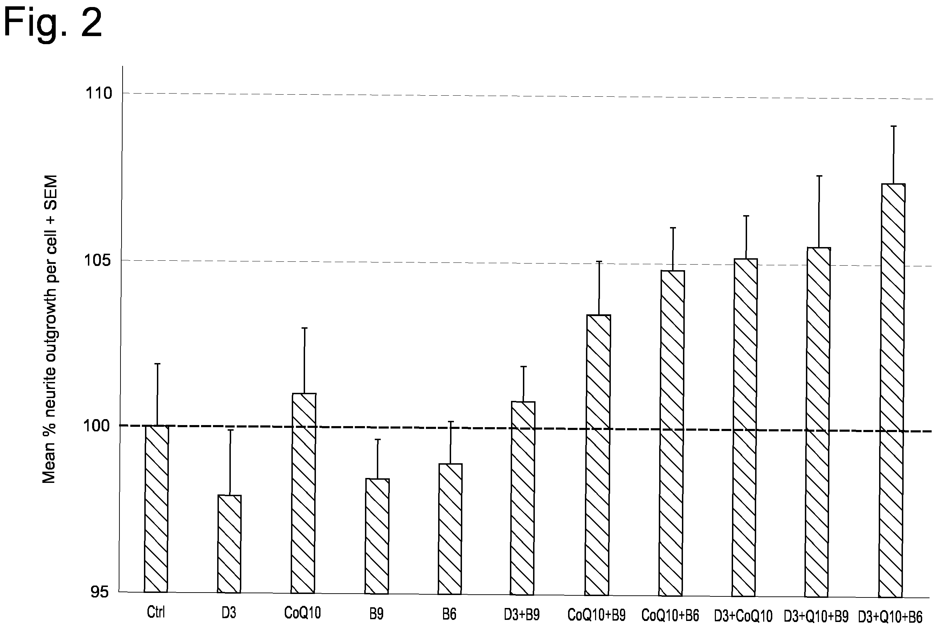

Image analysis of the neurite outgrowth after hypoxia showed that supplementation of the nutrients alone, did not affect neurite outgrowth as compared to control. However, when vitamin D3 and coenzyme Q10 were combined, an increased neurite outgrowth was observed relative to control. Similar results are observed when co-enzyme Q10 is combined with either vitamin B9 or vitamin B6. When combination of three are made, the neurite outgrowth is even more increased compared to the single and double combinations of nutrients, this effect is the highest when combining vitamin D3, coenzyme Q10 and vitamin B6. These findings are depicted in Figure 2 (vitamin D3 + co-enzyme Q10 + vitamin B9 or vitamin B6).

### Example 5 - Neurite outgrowth with co-enzyme Q10, vitamin D3, vitamin B2, PUFAs and a source of choline

The effects of nutrient supplementation on neurite outgrowth parameters in PC12 pheochromocytoma cells was also assessed. PC12 neurite outgrowth is one of the most widely used cell models for the study of neuronal differentiation.

An increase in the intensity of neurite outgrowth, is likely to have a positive effect in supporting brain functional connectivity and to contribute to a recovery of function in particular after a stroke. Any improvement in neurite outgrowth as observed in the PC12 cell study is likely to result in an improvement in those parts of the brain that were affected by the stroke.

### Materials and Method

PC12 pheochromocytoma cells were grown in DMEM (Gibco), supplemented with 10% fetal bovine serum (FBS), penicillin (100 units/ml), and streptomycin (100 µg/ml) (Gibco), under a humidified atmosphere with 95% air and 5% CO2 at 37°C. Cells were seeded in collagen (0.01 mg/ml) coated 96 well plates at a density of 5000 cells/well and after 24 hours supplemented with medium containing 20 ng/ml nerve growth factor (NGF) with or without coenzyme Q10 (1 µM), vitamin D3 ((0.25 µM), vitamin B2 (0.5 µM), DHA (6.7 µM), a source of choline (20 µM phosphatidylcholine, PC) or a mixture thereof.

In the PC12 experiments the cells were not exposed to hypoxia.

Docosahexaenoic acid (DHA) was resuspended in absolute ethanol and diluted 5 times in fetal bovine serum. All compounds were purchased from Sigma (Zwijndrecht, The Netherlands).

Supplementation of cells was performed in 6-fold in 96 wells plates. These conditions were compared to non-supplemented cells. After supplementation for 48 hours with these combinations, cells were stained using Calcein-AM stain (2 ng/µl) and the nuclei were counterstained using Hoechst stain (0.6 µg /ml), in 100 µl culture medium per well and incubated for 20 min at 37°C. Subsequently, FITC and DAPI images (9 images per well for 96 wells plate) were taken using an ImageXpress high content imaging system. Neurite length was quantified using the neurite outgrowth module in MetaXpress.

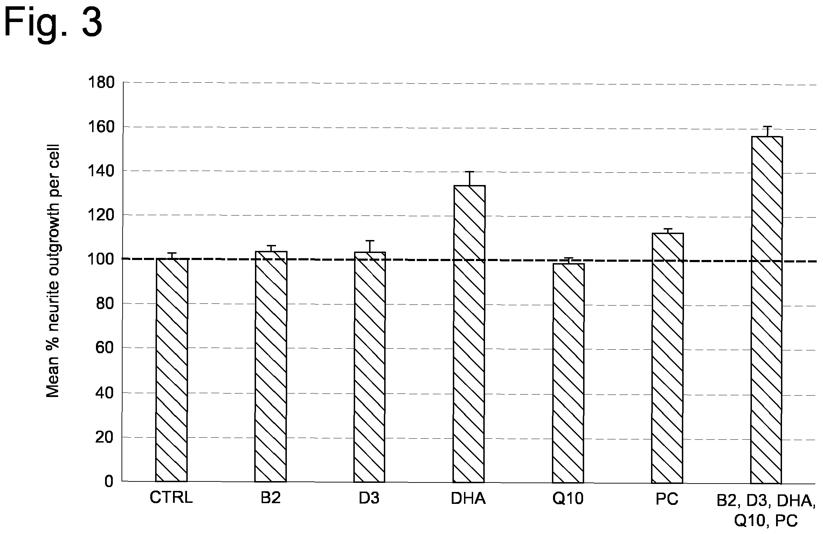

When co-enzyme Q10, vitamin D3, vitamin B2, DHA and PC were combined, an increased neurite outgrowth was observed relative to control (set at 100%). The observed neurite outgrowth with the combination of all 5 assessed compound resulted in a more than additive effect on neurite outgrowth as compared to the control (mean calculated or expected neurite outgrowth was 152, the observed mean neurite outgrowth of 157). The findings are depicted in Figure 3.

The combination of 5 ingredients in the PC12 model provided a synergistic increase in neurite outgrowth of these cells.

### Example 6 - Neurite outgrowth with choline and/or phosphatidyl choline

A similar experiment as described above in example 5 was performed wherein the effect of different ratio's of choline in the form of choline chloride and phosphatidylcholine at a similar total concentration of choline per condition on neurite outgrowth were assessed as compared to unsupplemented control PC12 cells (set at 100%).

Nutrients were supplemented alone (100% chol-Cl or 100% PC) and in a ratio of 60:40 and 40:60 of chol-Cl to PC. Supplementation of cells was performed in 6-fold in 96 wells plates at a total concentration of 20 µM choline, derived from chol-Cl, from PC, or from both. The neurite outgrowth in these conditions were compared to non-supplemented PC12 cells.

### Results

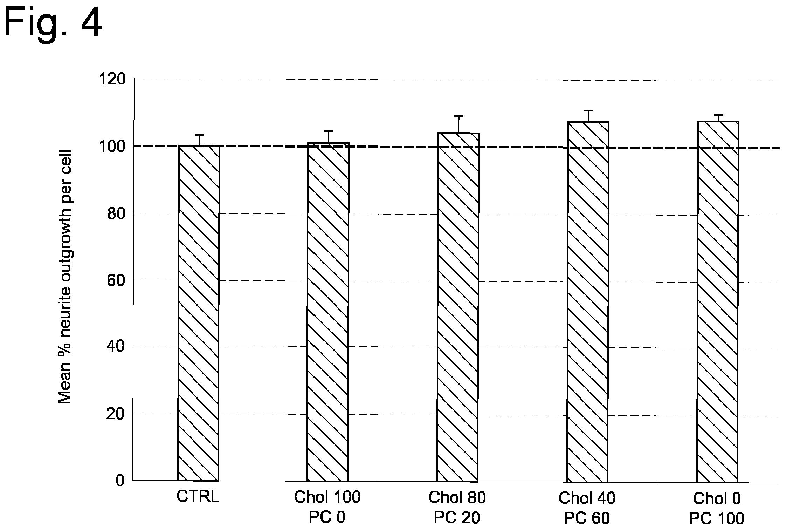

Image analysis of the neurite outgrowth showed that supplementation with 100% PC increases neurite outgrowth slightly more than supplementation with 100% chol-Cl as compared to the neurite outgrowth of the unsupplemented control PC12 cells. At a ratio of chol-Cl : PC of between 60:40 and 40:60 showed similar improved neurite outgrowth as PC12 cells supplemented with 100% chol-Cl. These findings are depicted in Figure 4.

The results suggest that choline provided in the form of PC may have a slightly better effect on neurite outgrowth than choline chloride at the same concentration of choline. Comparable effects to the 100% PC condition were obtained using mixtures with at least 40% of PC.

## Claims

1. A nutritional composition comprising a combination of (i) co-enzyme Q10, (ii) vitamin D, (iii) vitamin B2, (iv) polyunsaturated fatty acids and (v) choline, for use in the treatment of stroke, preferably ischemic stroke.

2. The nutritional composition for use according to claim 1 wherein the composition comprises (iv) polyunsaturated fatty acids selected from DHA, EPA and/or DPA.

3. The nutritional composition for use according to any one of claims 1 and 2 wherein choline is provided in the form of free choline, a choline salt and/or choline ester, wherein the choline ester is preferably phosphatidyl choline.

4. The nutritional composition for use according to any one of claims 1 to 3 wherein the composition further comprises one or more of (vi) vitamin B6 and (vii) vitamin B9.

5. The nutritional composition for use according to any one of claims 1 to 4 wherein the treatment of stroke comprises treating and/or preventing the consequences associated with said stroke in a subject that had a stroke.

6. The nutritional composition for use according to the any one of preceding claims wherein the treatment of stroke comprises an increase in length of neuronal neurite outgrowth in subjects suffering from stroke and treated with the composition as compared with subjects suffering from stroke and not treated with the composition.

7. The nutritional composition for use according to any one of the preceding claims wherein the treatment of stroke comprises the prevention of or the improvement of functional consequences associated with stroke selected from any one of hemiplegia, hemi-anaesthesia, language or visuo-spatial deficits and wherein the improvement is in a subject suffering from stroke-associated functional consequences and treated with the composition as compared with subjects suffering stroke and not treated with the composition.

8. The nutritional composition for use according to any one of the preceding claims wherein the composition is used for at least 2 weeks, preferably at least 4 weeks after diagnosis of the stroke.

9. The nutritional composition for use according to any one of the preceding claims wherein the composition comprises daily dosages of:
(i) 8 to 80 mg, preferably 10 to 70 mg, more preferably 15 to 60 mg co-enzyme Q10;
(ii) 6 to 75 µg, preferably 8 to 70 µg, more preferably 10 to 65 µg. vitamin D, preferably vitamin D3;
(iii) 0.8 to 7.6 mg, preferably 1 to 7.4 mg, more preferably in the range of 1.2 to 7.2 mg vitamin B2 and optionally
(iv) 150 to 1200 mg, preferably 200 to 1100 mg, more preferably 250 to 1000 mg DHA; and/or
300 to 2400 mg, preferably 400 to 2200 mg, more preferably 500 to 2000 mg per day EPA; and/or
(v) 200 to 1400 mg, preferably 300 to 1200 mg, more preferably 350 to 1100 mg choline.

10. The nutritional composition for use according to any one of the preceding claims further comprising one of more selected from A, C, and E vitamin(s), phospholipids, selenium, magnesium, and further B vitamins.

11. The nutritional composition for use according to any one of the preceding claims wherein the composition comprises vₐ) phosphatidylcholine and v_{b}) choline or a salt thereof, and wherein the weight to weight ratio phosphatidylcholine to choline is more than 0.1.

12. The nutritional composition for use according to the preceding claims wherein the nutritional composition is a liquid nutritional composition.

13. The nutritional composition for use according to the preceding claims wherein the nutritional composition is an oral nutritional supplement or a tube feed.

14. Liquid nutritional composition comprising
per 100 kcal
(i) 1.5 to 5.5 mg co-enzyme Q10;
(ii) 1.3 to 4.5 ug vitamin D, preferably vitamin D3;
(iii) 0.4 to 0.6 mg vitamin B2;
(v) 100 to 1000 mg, preferably 200 to 800 mg, more preferably 300 to 500 mg choline, choline salts and/or choline esters, wherein the composition comprises phosphatidylcholine and choline. and wherein the weight to weight ratio phosphatidylcholine to choline is more than 0.1;
optionally further comprising
(iv) 20 to 100 mg, preferably 25 to 90 mg, more preferably 30 to 80 mg DHA; and/or
20 to 225 mg, preferably 60 to 200 mg, more preferably 70 to 170 mg EPA;
and optionally at least one of (vi) and (vii);
(vi) 0.1 to 0.5 mg vitamin B6;
(vii) 16 to 80 µg vitamin B9.

15. Liquid nutritional composition according to claim 14 wherein the composition further comprises one of more selected from A, further B, C, E and K vitamin(s), phospholipids, selenium and magnesium.

16. Liquid nutritional composition according to any one of claims 14 and 15 wherein the composition further comprises per 100 kcal one or more of
(viii) 60 to 150 µg, Retinol Equivalents of vitamin A;
(ix) 0.05 to 0.4 mg vitamin B1;
(x) 0.4 to 1.4 µg vitamin B12;
(xi) 12 to 40 mg vitamin C;
(xii) 1 to 5.5 mg alpha-TE;
(xiii) 4 to 18 µg selenium; and,
(xiv) 10 to 70 mg magnesium.

17. Liquid nutritional composition according to any one of claims 14 to 16 wherein the composition further comprises per 100 kcal one or more of
(xv) 1 to 2.5 mg vitamin B3;
(xvi) 0.2 to 1 mg vitamin B5;
(xvii) 1.5 to 6 mg vitamin B7.

18. Liquid nutritional composition according to any one of claims 14 to 17, wherein the composition has a caloric density between 1.0 and 2.5 kcal per ml, preferably 1.2 and 2.4 kcal per ml.

19. Liquid nutritional composition according to claims 14 to 18 wherein the composition 4 to 7g protein per 100 kcal, 3 to 5.5 g lipids per 100 kcal and 8.5 to 10.5g carbohydrates per 100 kcal.

## Patentansprüche

1. Nährstoffzusammensetzung, umfassend eine Kombination aus (i) Coenzym Q10, (ii) Vitamin D, (iii) Vitamin B2, (iv) mehrfach ungesättigten Fettsäuren und (v) Cholin, zur Anwendung bei der Behandlung von Schlaganfällen, vorzugsweise ischämischen Schlaganfällen.

2. . Nährstoffzusammensetzung zur Anwendung nach Anspruch 1, wobei die Zusammensetzung (iv) mehrfach ungesättigte Fettsäuren umfasst, ausgewählt aus DHA, EPA und/oder DPA.

3. . Nährstoffzusammensetzung zur Anwendung nach einem der Ansprüche 1 und 2, wobei Cholin in Form von freiem Cholin, einem Cholinsalz und/oder einem Cholinester bereitgestellt wird, wobei der Cholinester vorzugsweise Phosphatidylcholin ist.

4. . Nährstoffzusammensetzung zur Anwendung nach einem der Ansprüche 1 bis 3, wobei die Zusammensetzung weiter eines oder mehrere von (vi) Vitamin B6 und (vii) Vitamin B9 umfasst.

5. . Nährstoffzusammensetzung zur Anwendung nach einem der Ansprüche 1 bis 4, wobei die Behandlung von Schlaganfällen die Behandlung und/oder Vorbeugung der mit dem Schlaganfall verbundenen Folgen bei einem Subjekt umfasst, das einen Schlaganfall erlitten hat.

6. . Nährstoffzusammensetzung zur Anwendung nach einem der voranstehenden Ansprüche, wobei die Behandlung eines Schlaganfalls eine Verlängerung des Auswachsens neuronaler Neuriten bei Subjekten umfasst, die einen Schlaganfall erlitten haben und mit der Zusammensetzung behandelt wurden, im Vergleich zu Subjekten, die einen Schlaganfall erlitten haben und nicht mit der Zusammensetzung behandelt wurden.

7. . Nährstoffzusammensetzung zur Anwendung nach einem der voranstehenden Ansprüche, wobei die Behandlung des Schlaganfalls die Vorbeugung oder Verbesserung von mit einem Schlaganfall verbundenen funktionellen Folgen umfasst, ausgewählt aus Hemiplegie, Hemianästhesie, Sprach- oder visuell-räumlichen Defiziten, und wobei die Verbesserung bei einem Subjekt auftritt, das an den mit dem Schlaganfall verbundenen funktionellen Folgen leidet und mit der Zusammensetzung behandelt wurde, im Vergleich zu Subjekten, die einen Schlaganfall erlitten haben und nicht mit der Zusammensetzung behandelt wurden.

8. . Nährstoffzusammensetzung zur Anwendung nach einem der voranstehenden Ansprüche, wobei die Zusammensetzung für mindestens 2 Wochen, vorzugsweise mindestens 4 Wochen nach der Diagnose des Schlaganfalls verwendet wird.

9. . Nährstoffzusammensetzung zur Anwendung nach einem der voranstehenden Ansprüche, wobei die Zusammensetzung Tagesdosen umfasst von:
(i) 8 bis 80 mg, vorzugsweise 10 bis 70 mg, noch bevorzugter 15 bis 60 mg Coenzym Q10;
(ii) 6 bis 75 µg, vorzugsweise 8 bis 70 µg, noch bevorzugter 10 bis 65 µg Vitamin D, vorzugsweise Vitamin D3;
(iii) 0,8 bis 7,6 mg, vorzugsweise 1 bis 7,4 mg, noch bevorzugter im Bereich von 1,2 bis 7,2 mg Vitamin B2 und optional
(iv) 150 bis 1200 mg, vorzugsweise 200 bis 1100 mg, noch bevorzugter 250 bis 1000 mg DHA; und/oder
300 bis 2400 mg, vorzugsweise 400 bis 2200 mg, noch bevorzugter 500 bis 2000 mg EPA pro Tag;
und/oder
(v) 200 bis 1400 mg, vorzugsweise 300 bis 1200 mg, noch bevorzugter 350 bis 1100 mg Cholin.

10. . Nährstoffzusammensetzung zur Anwendung nach einem der voranstehenden Ansprüche, weiter umfassend eines oder mehrere ausgewählt aus A-, C- und E-Vitaminen, Phospholipiden, Selen, Magnesium und weiteren B-Vitaminen.

11. . Nährstoffzusammensetzung zur Anwendung nach einem der voranstehenden Ansprüche, wobei die Zusammensetzung vₐ) Phosphatidylcholin und v_{b}) Cholin oder ein Salz davon umfasst und wobei das Gewichtsverhältnis von Phosphatidylcholin zu Cholin mehr als 0,1 beträgt.

12. . Nährstoffzusammensetzung zur Anwendung nach einem der voranstehenden Ansprüche, wobei die Nährstoffzusammensetzung eine flüssige Nährstoffzusammensetzung ist.

13. . Nährstoffzusammensetzung zur Anwendung nach einem der voranstehenden Ansprüche, wobei die Nährstoffzusammensetzung ein orales Nahrungsergänzungsmittel oder ein Sondennahrungsmittel ist.

14. . Flüssige Nährstoffzusammensetzung, umfassend
pro 100 kcal
(i) 1,5 bis 5,5 mg Coenzym Q10;
(ii) 1,3 bis 4,5 ug Vitamin D, vorzugsweise Vitamin D3;
(iii) 0,4 bis 0,6 mg Vitamin B2;
(v) 100 bis 1000 mg, vorzugsweise 200 bis 800 mg, noch bevorzugter 300 bis 500 mg Cholin, Cholinsalze und/oder Cholinester, wobei die Zusammensetzung Phosphatidylcholin und Cholin umfasst.
und wobei das Gewichtsverhältnis von Phosphatidylcholin zu Cholin mehr als 0,1 beträgt;
optional weiter umfassend
(iv) 20 bis 100 mg, vorzugsweise 25 bis 90 mg, noch bevorzugter 30 bis 80 mg DHA; und/oder
20 bis 225 mg, vorzugsweise 60 bis 200 mg, noch bevorzugter 70 bis 170 mg EPA;
und optional mindestens eines von (vi) und (vii);
(vi) 0,1 bis 0,5 mg Vitamin B6;
(vii) 16 bis 80 µg Vitamin B9.

15. . Flüssige Nährstoffzusammensetzung nach Anspruch 14, wobei die Zusammensetzung weiter eines oder mehrere umfasst ausgewählt aus Vitamin A, weiteren B-Vitaminen, Vitamin C, Vitamin E und Vitamin K, Phospholipiden, Selen und Magnesium.

16. . Flüssige Nährstoffzusammensetzung nach einem der Ansprüche 14 und 15, wobei die Zusammensetzung weiter pro 100 kcal eines oder mehrere umfasst von
(viii) 60 bis 150 µg Retinol-Äquivalenten von Vitamin A;
(ix) 0,05 bis 0,4 mg Vitamin B1;
(x) 0,4 bis 1,4 µg Vitamin B12;
(xi) 12 bis 40 mg Vitamin C;
(xii) 1 bis 5,5 mg Alpha-TE;
(xiii) 4 bis 18 µg Selen; und
(xiv) 10 bis 70 mg Magnesium.

17. . Flüssige Nährstoffzusammensetzung nach einem der Ansprüche 14 bis 16, wobei die Zusammensetzung pro 100 kcal weiter eines oder mehrere umfasst von
(xv) 1 bis 2,5 mg Vitamin B3;
(xvi) 0,2 bis 1 mg Vitamin B5;
(xvii) 1,5 bis 6 mg Vitamin B7.

18. . Flüssige Nährstoffzusammensetzung nach einem der Ansprüche 14 bis 17, wobei die Zusammensetzung eine Kaloriendichte zwischen 1,0 und 2,5 kcal pro mL, vorzugsweise zwischen 1,2 und 2,4 kcal pro mL, aufweist.

19. . Flüssige Nährstoffzusammensetzung nach den Ansprüchen 14 bis 18, wobei die Zusammensetzung 4 bis 7 g Protein pro 100 kcal, 3 bis 5,5 g Lipide pro 100 kcal und 8,5 bis 10,5 g Kohlenhydrate pro 100 kcal enthält.

## Revendications

1. Composition nutritionnelle comprenant une combinaison (i) de coenzyme Q10, (ii) de vitamine D, (iii) de vitamine B2, (iv) d'acides gras polyinsaturés et (v) de choline, destinée à être utilisée dans le traitement d'accidents vasculaires cérébraux, de préférence d'accidents vasculaires cérébraux ischémiques.

2. Composition nutritionnelle destinée à être utilisée selon la revendication 1, où la composition comprend (iv) des acides gras polyinsaturés choisis parmi le DHA, l'EPA et/ou le DPA.

3. Composition nutritionnelle destinée à être utilisée selon l'une quelconque des revendications 1 et 2, où la choline est fournie sous forme de choline libre, d'un sel de choline et/ou d'un ester de choline, où l'ester de choline est de préférence la phosphatidylcholine.

4. Composition nutritionnelle destinée à être utilisée selon l'une quelconque des revendications 1 à 3, où la composition comprend en outre une ou plusieurs des vitamines suivantes : (vi) vitamine B6 et (vii) vitamine B9.

5. Composition nutritionnelle destinée à être utilisée selon l'une quelconque des revendications 1 à 4, où le traitement d'un accident vasculaire cérébral comprend le traitement et/ou la prévention des conséquences associées audit accident vasculaire cérébral chez un sujet qui a subi un accident vasculaire cérébral.

6. Composition nutritionnelle destinée à être utilisée selon l'une quelconque des revendications précédentes, où le traitement d'un accident vasculaire cérébral comprend une augmentation de la longueur de l'excroissance des neurites neuronaux chez des sujets souffrant d'un accident vasculaire cérébral et traités avec la composition par rapport aux sujets souffrant d'un accident vasculaire cérébral et non traités avec la composition.

7. Composition nutritionnelle destinée à être utilisée selon l'une quelconque des revendications précédentes, où le traitement d'un accident vasculaire cérébral comprend la prévention ou l'amélioration des conséquences fonctionnelles associées à l'accident vasculaire cérébral, choisies parmi l'hémiplégie, l'hémi-anesthésie, les déficits du langage ou visuo-spatiaux, et où l'amélioration concerne un sujet souffrant de conséquences fonctionnelles associées à un accident vasculaire cérébral et traité avec la composition, par rapport à des sujets souffrant d'un accident vasculaire cérébral et non traités avec la composition.

8. Composition nutritionnelle destinée à être utilisée selon l'une quelconque des revendications précédentes, où la composition est utilisée pendant au moins 2 semaines, de préférence au moins 4 semaines après le diagnostic de l'accident vasculaire cérébral.

9. Composition nutritionnelle destinée à être utilisée selon l'une quelconque des revendications précédentes, où la composition comprend des doses quotidiennes de :
(i) 8 à 80 mg, de préférence 10 à 70 mg, plus préférablement 15 à 60 mg de coenzyme Q10 ;
(ii) 6 à 75 µg, de préférence 8 à 70 µg, plus préférablement 10 à 65 µg de vitamine D, de préférence de vitamine D3 ;
(iii) 0,8 à 7,6 mg, de préférence 1 à 7,4 mg, plus préférablement dans la plage de 1,2 à 7,2 mg de vitamine B2 et éventuellement
(iv) 150 à 1200 mg, de préférence 200 à 1100 mg, plus préférablement 250 à 1000 mg de DHA ; et/ou
300 à 2400 mg, de préférence 400 à 2200 mg, plus préférablement 500 à 2000 mg par jour d'EPA ; et/ou
(v) 200 à 1400 mg, de préférence 300 à 1200 mg, plus préférablement 350 à 1100 mg de choline.

10. Composition nutritionnelle destinée à être utilisée selon l'une quelconque des revendications précédentes, comprenant en outre un ou plusieurs éléments choisis parmi les vitamines A, C et E, les phospholipides, le sélénium, le magnésium et d'autres vitamines B.

11. Composition nutritionnelle destinée à être utilisée selon l'une quelconque des revendications précédentes, où la composition comprend vₐ) de la phosphatidylcholine et v_{b}) de la choline ou un sel de celle-ci, et où le rapport pondéral entre la phosphatidylcholine et la choline est supérieur à 0,1.

12. Composition nutritionnelle destinée à être utilisée selon les revendications précédentes, où la composition nutritionnelle est une composition nutritionnelle liquide.

13. Composition nutritionnelle destinée à être utilisée selon les revendications précédentes, où la composition nutritionnelle est un complément nutritionnel oral ou une alimentation par sonde.

14. Composition nutritionnelle liquide comprenant
pour 100 kcal
(i) 1,5 à 5,5 mg de coenzyme Q10 ;
(ii) 1,3 à 4,5 µg de vitamine D, de préférence de vitamine D3 ;
(iii) 0,4 à 0,6 mg de vitamine B2 ;
(v) 100 à 1000 mg, de préférence 200 à 800 mg, plus préférablement 300 à 500 mg de choline, de sels de choline et/ou d'esters de choline, où la composition comprend de la phosphatidylcholine et de la choline.
et où le rapport pondéral entre la phosphatidylcholine et la choline est supérieur à 0,1 ;
comprenant éventuellement en outre
(iv) 20 à 100 mg, de préférence 25 à 90 mg, plus préférablement 30 à 80 mg de DHA ; et/ou
20 à 225 mg, de préférence 60 à 200 mg, plus préférablement 70 à 170 mg d'EPA ;
et éventuellement au moins l'un parmi (vi) et (vii) ;
(vi) 0,1 à 0,5 mg de vitamine B6 ;
(vii) 16 à 80 µg de vitamine B9.

15. Composition nutritionnelle liquide selon la revendication 14, où la composition comprend en outre un ou plusieurs éléments choisis parmi les vitamines A, B, C, E et K, les phospholipides, le sélénium et le magnésium.

16. Composition nutritionnelle liquide selon l'une quelconque des revendications 14 et 15, où la composition comprend en outre, pour 100 kcal, un ou plusieurs des éléments suivants
(viii) 60 à 150 µg d'équivalents rétinol de vitamine A ;
(ix) 0,05 à 0,4 mg de vitamine B1 ;
(x) 0,4 à 1,4 µg de vitamine B12 ;
(xi) 12 à 40 mg de vitamine C ;
(xii) 1 à 5,5 mg d'alpha-TE ;
(xiii) 4 à 18 µg de sélénium ; et
(xiv) 10 à 70 mg de magnésium.

17. Composition nutritionnelle liquide selon l'une quelconque des revendications 14 à 16, où la composition comprend en outre, pour 100 kcal, un ou plusieurs des éléments suivants
(xv) 1 à 2,5 mg de vitamine B3 ;
(xvi) 0,2 à 1 mg de vitamine B5 ;
(xvii) 1,5 à 6 mg de vitamine B7.

18. Composition nutritionnelle liquide selon l'une quelconque des revendications 14 à 17, où la composition a une densité calorique comprise entre 1,0 et 2,5 kcal par ml, de préférence entre 1,2 et 2,4 kcal par ml.

19. Composition nutritionnelle liquide selon les revendications 14 à 18, où la composition contient 4 à 7 g de protéines pour 100 kcal, 3 à 5,5 g de lipides pour 100 kcal et 8,5 à 10,5 g de glucides pour 100 kcal.
